(19) Europäisches Patentamt
European Patent Office
Office européen des brevets

(11) EP 1 440 680 A1

(12) # EUROPEAN PATENT APPLICATION
published in accordance with Art. 158(3) EPC

(43) Date of publication:
**28.07.2004 Bulletin 2004/31**

(21) Application number: **02798830.2**

(22) Date of filing: **12.09.2002**

(51) Int Cl.[7]: **A61K 7/00**, A61K 7/06, A61K 7/04, C08F 293/00

(86) International application number:
**PCT/JP2002/009338**

(87) International publication number:
**WO 2003/024414 (27.03.2003 Gazette 2003/13)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **13.09.2001 JP 2001277521**
**29.03.2002 JP 2002093943**
**21.05.2002 JP 2002145976**
**28.05.2002 JP 2002154294**
**30.05.2002 JP 2002156777**
**19.07.2002 JP 2002211360**
**22.07.2002 JP 2002212443**

(71) Applicant: **Mitsubishi Chemical Corporation**
**Tokyo 108-0014 (JP)**

(72) Inventors:
- **HIWATASHI, Tomoaki, c/o MITSUBISHI CHEMICAL CORP. Yokkaichi-shi, Mie 510-8530 (JP)**
- **SHIBATA, Minako, c/o MITSUBISHI CHEMICAL CORP. Yokkaichi-shi, Mie 510-8530 (JP)**
- **NISHIZAWA, Osamu, c/o MITSUBISHI CHEMICAL CORP. Yokkaichi-shi, Mie 510-8530 (JP)**
- **ONOE, Masato, c/o MITSUBISHI CHEMICAL CORPORATION Yokkaichi-shi, Mie 510-8530 (JP)**
- **KITANI, Yasuo, c/o MITSUBISHI CHEMICAL CORPORATION Yokkaichi-shi, Mie 510-8530 (JP)**

(74) Representative:
**Meyers, Hans-Wilhelm, Dr.Dipl.-Chem. et al**
**Patentanwälte**
**von Kreisler-Selting-Werner**
**Postfach 10 22 41**
**50462 Köln (DE)**

(54) **COSMETIC POLYMER COMPOSITION AND COSMETIC**

(57) The present invention discloses a cosmetic polymer composition containing a straight-chain block copolymer, where the copolymer having a unit derived from a compound having an ethylenic unsaturated bond, having a number-average molecular weight of $1.0\times10^3$ to $1.0\times10^6$, and having at least two or more glass transition points or melting points; a hair cosmetic polymer composition containing a copolymer a copolymer capable of forming a film having a Young's modulus of 50 MPa or larger and a fracture-point elongation of 100% or larger, and dispersible into water and/or alcohol; and a cosmetic material containing these compositions.

EP 1 440 680 A1

**Description**

Field of the Invention

The present invention relates to cosmetic polymer compositions and cosmetics using the same, containing a copolymer having specific properties, and in particular containing a straight-chain block copolymer, and more specifically relates to cosmetic polymer compositions and cosmetics using the same, containing a block copolymer having two or more units, which are introduced into the molecular skeleton thereof as block-wise unit, having different properties each other.

Background Art

There are known cosmetic materials containing a film-forming polymer, intended for use on skin, nail and hair. For example, for the purpose of improving makeup-lasting property, protecting skin or nail, or coloring or decorating skin or nail, film-forming polymers have been used in those for skin and nail. For the purpose of improving affinities to hairs or adhesiveness between hairs, and thereby having hair-style-keeping property, film-forming polymers have been used in those for hair. Examples of the polymer conventionally used for hair cosmetics include nonionic-base, anionic-base, cationic base and amphoteric-base ones. Although, as nonionic-base polymer, poly (vinyl methyl ether) or polyvinylpyrrolidone has been used, properties of polyvinypyrrolidone are highly influenced by humidity conditions, and properties of a film formed of such a polymer are therefore also influenced by humidity conditions. More specifically, on one hand, the film before being moistened is hard and thus causative of flaking, but on the other hand, the film is considerably softened under a hot and humid environment so as to cause blocking, and tends to make it difficult to comb or brush the hair due to adhesion of hairs. Properties of poly(vinyl methyl ether) are further significantly influenced by humidity condition.

Examples of the anionic-base polymer include a copolymer of vinylcarboxylic acid having a carboxylic acid group as an anionic group (e.g., acrylic acid, methacrylic acid) and styrene or alkyl acrylic ester. Unlike the nonionic-base polymer, properties of the anionic-base polymer are less influenced by susceptible to humidity, but has a poor affinity to hair because of its anionic property as same as hair. Although the anionic-base polymer film is hard and excellent in hair styling property, on the other hand, it is rather brittle and highly causative of flaking. Moreover, its anionic property limits addition of any cationic substances, so that solidification typically due to rinse material (cationic) during hair wash is also anticipated.

The cationic-base polymer has larger affinity to hair as compared with that of two aforementioned polymers, but its properties are influenced by humidity conditions as well as properties of the nonionic-polymer. Its cationic property also raises anticipation of toxicity or skin irritation property, limitation of addition of any anionic-base substances, and solidification caused by shampoo (anionic) during hair wash.

Recently, there has been a need for alcohol-free hair cosmetic because of its merit of easy removal by hair wash, readiness in preparation of hair cosmetic simply by dilution with water, and environmental friendliness. From this point of view, a polymer used for the hair cosmetic is preferably of water soluble. There is, however, a general problem that improving in water solubility of polymer contributes to lowering setting power. To overcome these drawbacks in the hair cosmetic materials containing the anionic-base polymer, cationic-base polymer and nonionic-base polymer, there are proposed cosmetic materials using an amphoteric polymer and polarized polymer which are copolymers having carboxybetaine or amine oxide as a hydrophilic group (see Japanese Laid-Open Patent Publication Nos. syo 51-9732, syo 55-104209 and hei 10-72323). The amphoteric polymer and polarized polymer are, however, disadvantageous in having only an insufficient compatibility with various cosmetic base materials.

The aforementioned general-purpose ionic polymers have played a role of keeping hair styling through affinity to hair, film-formation property and adhesion property between hair yarns, while exhibiting the individual features. The styling materials using these general-purpose ionic polymers, however, suffer from a problem of tacky feeling under a moistened condition, and stiff feeling under a dried condition. For actual prescription, in order to improve such feelings, a small amount of oil is added to the polymer. Addition of the oil material, however, lowers ability of the polymer and makes it difficult to keep its intrinsically excellent setting ability. This raises a strong demand for developing a new polymer capable of keeping a plurality of good abilities such as setting ability or ability to give nice feeling, and can readily be removed by hair wash.

Based on this situation, one investigation have been made on a thermoplastic elastomer having a random-copolymerized principal chain and water soluble graft chains, and being soluble or dispersible in water or alcohol. This is, however, still on the way to achieve polymer elasticity sufficient for used as a cosmetic polymer composition (Published Japanese Translations of PCT International Publication for Patent Publications No. hei 8-512083, Japanese Laid-Open Patent Publication No. hei 11-181029).

International Patent Publication WO98/51722 proposes a method of manufacturing a graft polymer using

living radical polymerization, and application of this method to production of personal care products. The method described in this publication is successful in obtaining a graft polymer having a controlled molecular weight and so forth in an efficient manner by adopting living polymerization. The above-described publication, however, only mentions a method of manufacturing graft polymer or branched polymer, and applications of the polymer, and does not mention any straight-chain block copolymer at all.

International Patent Publication WO00/40628 and U.S. Patent No. 6,410,005B1 respectively disclose hair styling compositions which contain block copolymers individually having a film-forming ability and at least two glass transition points. The block copolymers described in these publications are, however, branched block copolymers containing cross-linked structures, and there is no description on straight-chain block copolymer in this publication as well as in the aforementioned publication.

The straight-chain block copolymer has a desirable molecular flexibility as being expected from its structure, and supposedly has a larger elastic recovery force as compared with the branched block copolymer having the same composition.

Some thermoplastic elastomeric copolymers are known, and typical examples thereof widely used include styrene butadiene copolymer, styrene-isoprene copolymer and hydrogen-added product of these copolymers (respectively referred to as styrene-ethylene-butylene copolymer and styrene-ethylene-propylene copolymer). These block copolymers have both of thermoplastic-like property, which contributes to solubility and strength of the copolymer, and rubber-like elasticity which contributes to flexibility and shape-retaining property. These block copolymers are also known to be available as a compatibilizing agent for various general-purpose thermoplastics. Most of the thermoplastic elastomeric copolymer are, however, generally insoluble or less soluble in aqueous and/or alcoholic solvent, and are not suitable for the cosmetic polymer composition.

It should be useful in developing an improved cosmetic polymer composition if any thermoplastic elastomeric copolymer having the above-described properties and solubility in water and/or alcohol can be provided. It is also expected that use of the above-described, water-soluble thermoplastic elastomer as a compatibilizing agent for general-purpose resins breaks a way to a polymer blend (combined use of an anionic polymer and a cationic polymer), which has been unpractical.

Disclosure of the Invention

An object of the present invention is therefore to provide a cosmetic polymer composition and a cosmetic material capable of satisfying at the same time a plurality of abilities required for the cosmetic material by using a straight-chain block copolymer having two or more properties. Another object of the present invention is to provide a cosmetic material having an excellent film-forming ability on hair, skin or nail, flexibility ascribable to its elasticity, and excellent feeling in use without contributing to tacky feeling nor uncomfortable feeling due to the film formation, and is to provide also a cosmetic polymer composition capable of producing such cosmetic material.

One aspect of the present invention relates to a cosmetic polymer composition comprising a straight-chain block copolymer having a unit derived from a compound having an ethylenic unsaturated bond, having a number-average molecular weight of $1.0\times10^3$ to $1.0\times10^6$, and having two or more glass transition points or melting points.

Preferred embodiments of the present invention include:

the cosmetic polymer composition wherein the block copolymer comprises at least one block composed of a unit having a hydrophilic group;

the cosmetic polymer composition wherein the hydrophilic group is at least any one selected from groups consisting of an anionic group consisting of carboxylic acid group, sulfonic acid group, phosphonic acid group and salts of these groups; a cationic group consisting of amino group (including quaternary ammonium salt group), pyridyl group and salts of these groups; a nonionic group consisting of hydroxyl group, alkoxy group, epoxy group, amido group and cyano group; an amphoteric ionic group consisting of carboxybetaine group; and a semipolar group consisting of amine oxide group;

the cosmetic polymer composition wherein the block copolymer comprises at least one of units represented by formulae (1) to (5) below:

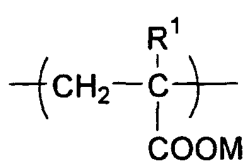

(1)

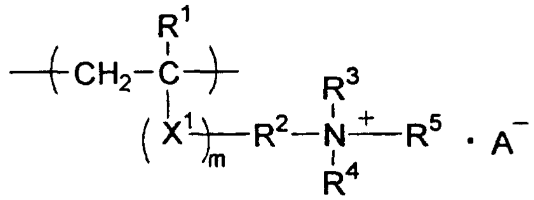

(2)

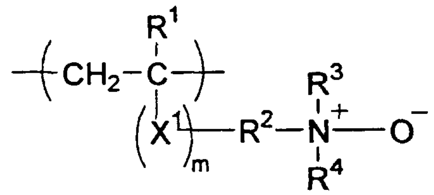

(3)

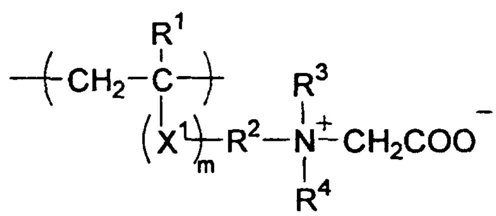

(4)

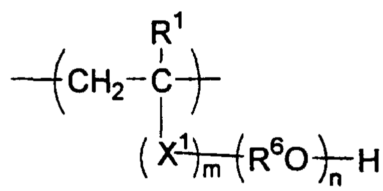

(5)

(where, $R^1$ represents a hydrogen atom or a methyl group; $R^2$ and $R^6$ respectively represent a $C_{1-4}$ straight-chain or branched alkylene group; $R^3$, $R^4$ and $R^5$ respectively represent a hydrogen atom, a $C_{1-24}$ alkyl group, a $C_{6-24}$ aryl group, or any combination thereof such as $C_{7-24}$ arylalkyl group or alkylaryl group; and $X^1$ represents -COO-, -CONH-, -O- or NH-. $A^-$ represents an anion; and M represents a hydrogen atom, an alkali metal ion or an ammonium ion. m is 0 or 1; and n is any integer from 1 to 50.);
the cosmetic polymer composition wherein the block copolymer comprises a unit derived from an ethylenic unsaturated carboxylic acid, and a unit derived from an ethylenic unsaturated carboxylate ester; the cosmetic polymer composition wherein the block copolymer comprises the unit derived from an ethylenic unsaturated carboxylic acid in an amount of 10 to 90% by weight, and the unit derived from an ethylenic unsaturated carboxylate ester in an amount of 90 to 10% by weight; and the cosmetic polymer composition wherein the block copolymer is a di-block copolymer, tri-block copolymer or multi-block copolymer.

Other preferred embodiments of the present invention include:

the cosmetic polymer composition wherein the block copolymer has a glass transition point or a melting point nearly equal to a glass transition point (occasionally referred to as "Tg", hereinafter) or a melting point (occasionally referred to as "Tm", hereinafter) of a homopolymer composed of the monomer which make up at least one block of the block copolymer;
the cosmetic polymer composition wherein a block unit composing the block copolymer has a number-average molecular weight of $1.0\times10^3$ to $1.0\times10^5$;
the cosmetic polymer composition wherein weight-average molecular weight (Mw) and number-average molecular weight (Mn) of the block copolymer give a ratio (Mw/Mn) of 2.5 or less; and
the cosmetic polymer composition wherein the block copolymer is dispersible or soluble in water and/or alcohol.

Still other preferred embodiments of the present invention include:

the cosmetic polymer composition wherein the block copolymer comprises a block formed by post-treatment after polymerization;

the cosmetic polymer composition wherein the post-treatment is hydrolysis;

the cosmetic polymer composition wherein the block copolymer is produced by controlled radical polymerization using an organic halide as an initiator, and using, as a catalyst, at least a metal complex having a metal selected from Group VIII, Group IX, Group X and Group XI elements in the periodic table as a central metal; and

the cosmetic polymer composition wherein the block copolymer is produced by controlled radical polymerization using a metal complex, derived from a copper halide and an amine compound, as a catalyst.

Still other preferred embodiments of the present invention include:

a hair cosmetic polymer composition comprising a copolymer capable of forming a film having a Young's modulus (measured according to JIS K7161 under a tensile speed of 20 mm/min) of 50 MPa or larger and a fracture-point elongation of 100% or larger, and dispersible into water and/or alcohol; and a hair cosmetic polymer composition comprising a copolymer capable of forming a film having a Young's modulus of 100 MPa or larger and a fracture-point elongation of 150% or larger, and dispersible into water and/or alcohol.

Other preferred embodiments of the present invention include the hair cosmetic polymer composition comprising, in addition to the copolymer, an anionic polymer, cationic polymer, nonionic polymer, amphoteric polymer, amine oxide-group-containing polymer or silicone derivative.

From other point of view, a cosmetic comprising the cosmetic polymer composition; and the cosmetic for use on hair, skin or nail, comprising such cosmetic polymer composition are provided by the present invention.

Best Modes for Carrying out the Invention

Next paragraphs will further detail the present invention. It is to be noted that, in the present specification, a word "to" indicates a range including the numerical values placed therebefore and thereafter as a minimum value and a maximum value.

I. Straight-Chain Block Copolymer

The cosmetic polymer composition of the present invention comprises a straight-chain block copolymer. It is to be noted that, in the present invention, "straight-chain" block copolymer means such copolymer comprising a polymer block chain composed of one unit, or two or more monomer units, and other polymer block chain composed of monomer unit different therefrom, an end portion of which being bonded to at least one end portion of the former block chain.

The block copolymer has a unit derived from a compound having an ethylenic unsaturated bond, and has a number-average molecular weight of $1.0\times10^3$ to $1.0\times10^6$. The number-average molecular weight of the block copolymer exceeding $1.0\times10^6$ may result in an increased viscosity of the solution, and may raise a problem in its use as a cosmetic material. On the other hand, the number-average molecular weight less than $1.0\times10^3$ will result in a degraded film-forming ability, and may fail in achieving a sufficient performance in its use as a cosmetic material. A desirable range of the weight-average molecular weight of the block copolymer varies depending on purposes of use, and preferably falls in a range from $5.0\times10^3$ to $3.0\times10^5$ for use as a hair cosmetic material, in particular as a hair styling agent, and in a range from $2.0\times10^4$ to $1.5\times10^5$ for use as an agent for supplying a conditioning function. For use as cosmetic materials to be applied on skin and nail, the preferable range falls in a range from $1.0\times10^4$ to $5\times10^5$, and more preferably from $2.0\times10^4$ to $1.0\times10^6$.

Although there is no specific limitation on the ratio (Mw/Mn) of weight-average molecular weight (Mw) to number-average molecular weight (Mn) of the block copolymer, measured by gel permeation chromatography, the ratio is preferably 2.5 or less, more preferably 2.0 or less, and still more preferably 1.8 or less. Mw/Mn exceeding 2.5 tends to degrade the uniformity of the block copolymer. The controlled radical polymerization process, described later, is successful in obtaining a uniform block copolymer having Mw/Mn typically as small as 2.0 or less, which can provide an excellent feel of touch when used as a cosmetic material.

Although there is no specific limitation on the number-average molecular weight of the individual block components of the block copolymer, it preferably falls in a range from $1.0\times10^3$ to $1.0\times10^5$, and more preferably from $5.0\times10^3$ to $1.0\times10^5$. The number-average molecular weight small than $1.0\times10^3$ may tend to excessively lower the viscosity, and on the other hand, the number-average molecular weight exceeding $1.0\times10^5$ may tend to excessively increase the viscosity, so that it is preferably set within a aforementioned range depending on required performances

of the polymer.

The block copolymer of the present invention has two or more glass transition points or melting points. Of two glass transition points, the higher one is preferably 25°C or higher, more preferably 40°C or higher, and still more preferably 50°C or higher. Of two glass transition points, the lower one is preferably lower than 25°C, more preferably 0°C or lower, and still more preferably -20°C or lower. All of the melting points are preferably 25°C or around, or higher. The block copolymer preferably has a glass transition point or a melting point attributed to each block, and in other words, preferably has a glass transition point or a melting point nearly equal to the glass transition point or the melting point of a homopolymer composed of a monomer which makes up one block of the block copolymer. For example, a block copolymer of A-B type preferably has two glass transition points or melting points, where each of the glass transition points or melting points is nearly equal to a glass transition point or a melting point individually attributed to homopolymers of A and B. On the other hand, a block copolymer of A-B-C type preferably has three glass transition points or melting points, where each of the glass transition points or melting points is nearly equal to a glass transition point or a melting point individually attributed to homopolymers of A, B and C.

Glass transition point (Tg) and melting point (Tm) refer to temperatures at which the polymer or a part thereof transits from a state of brittle material or solid into a state of rubber-like material or liquid, and can typically be measure by the differential scanning calorimetry. Glass transition phenomenon of polymer is described in "Introduction to Polymer Science and Technology", An SPE Textbook (eds. H. S. Kaufman and J.J. Falcetta), published by John Wiley & Sons (1977).

The following description deals with Tg, as a representative of Tg and Tm.

The block copolymer comprising at least one block having a hydrophilic group can readily be removed by hair wash, and is therefore preferably used for hair cosmetic material having an excellent hair washing property. The block having a hydrophilic group is also advantageous in allowing the block copolymer to be blended with cosmetic materials in various forms. The hydrophilic group may be any of anionic group, cationic group, nonionic group and amphoteric group. Examples of the anionic group include carboxylic acid group, sulfonic acid group, phosphonic acid group and salts of these groups; examples of the cationic group include amino group (including quaternary ammonium group), pyridyl group and salts of these groups; examples of the nonionic group include hydroxyl group, alkoxy group, epoxy group, amido group, cyano group; examples of the amphoteric ionic group include carboxybetaine group, sulfobetaine group and phosphobetaine group; and examples of the semipolar group include amine oxide group. These hydrophilic groups can be introduced into the block copolymer by polymerizing a monomer originally having the hydrophilic group, or by subjecting the copolymer after being polymerized to a post-treatment such as hydrolysis.

It is to be noted that the semipolar group in the context of this patent specification does not mean an apparent ionic group, but instead means a group having both features of ionic bond and covalent bond, and having a biased electron distribution.

The block copolymer has a unit derived from a compound having an ethylenic unsaturated bond. As described in the above, at least one block of the block copolymer preferably has a hydrophilic group. The unit having a hydrophilic group is preferably any one of units represented by the formulae (1) to (5) below:

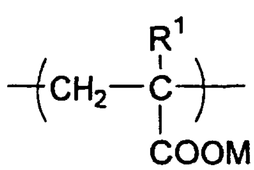

(1)

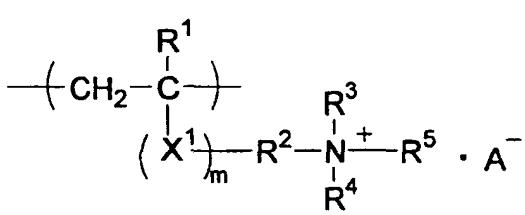

(2)

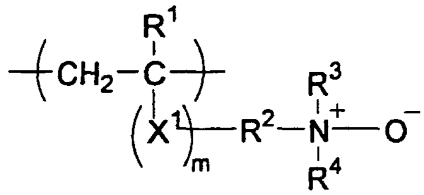

(3)

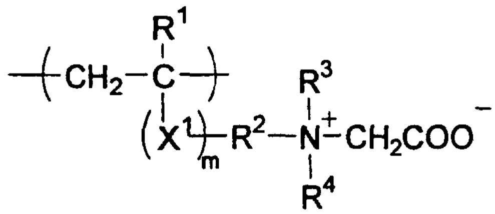

(4)

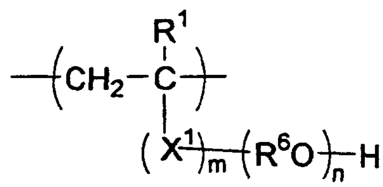

(5)

In the formulae, $R^1$ represents a hydrogen atom or a methyl group; $R^2$ and $R^6$ respectively represent a $C_{1-4}$ straight-chain or branched alkylene group; $R^3$, $R^4$ and $R^5$ respectively represent a hydrogen atom, a $C_{1-24}$ alkyl group, a $C_{6-24}$ aryl group, or any combination thereof such as $C_{7-24}$ arylalkyl group or alkylaryl group; and $X^1$ represents -COO-, -CONH-, -O- or NH-. $A^-$ represents an anion; and M represents a hydrogen atom, an alkali metal ion or an ammonium ion. m is 0 or 1; and n is any integer from 1 to 50.

The $C_{1-24}$ alkyl group respectively represented by $R^3$, $R^4$ and $R^5$ include all of straight-chain, branched-chain and cyclic alkyl groups. The same will apply also to the alkyl group portion of the arylalkyl group and the alkyl group portion of alkylaryl group represented respectively by $R^3$, $R^4$ and $R^5$.

Anion represented by $A^-$ is exemplified by anionic group of acid, and specific examples thereof include halogen ion, sulfate ion and $COO^-$. Alkali metal ion represented by M can be exemplified by $Na^+$ and $K^+$. Examples of ammonium ion represented by M not only include $NH_4^+$ derived from ammonia, but also include alkylammonium ions derived from amines such as volatile amines such as methylamine, dimethylamine, trimethylamine, ethylamine, diethylamine, triethylamine, n-propylamine, n-butylamine, allylamine, ethylenediamine, morpholine and pyridine; and non-volatile amines such as mono-, di- or triethanolamine, mono-, di- or triisopropanolamine, aminoethyl propanol, aminoethyl propanediol and lysine.

The block containing unit represented by any of the foregoing formulae (1) to (5) can be manufactured by using, as a monomer, a double-bond-containing compound corresponded to any of the units represented by the formulae (1) to (5). Even for the case where the compounds corresponded to the units represented by the formulae (1) to (5) are not used, the block can be manufactured by polymerizing other monomer and subjecting the product to post-treatment such as hydrolysis. For example, a block copolymer having a block composed of the unit represented by the formula (1) can be manufactured also by using (meth)acrylic ester as a copolymerization monomer, in which a copolymer having a block composed of such monomer is synthesized, and the obtained block is then hydrolyzed (and is further neutralized if necessary). The block copolymer may be such as having two or more species of the blocks composed of the unit represented by any of the formulae (1) to (5).

Examples of the compounds having ethylenic unsaturated bond, capable of composing the block copolymer, (including examples of the compounds capable of forming the units represented by the formulae (1) to (5)) are listed below, where the present invention is by no means limited by these specific examples.

Specific examples of the nonionic monomers:

Acrylic esters such as unsaturated methyl acrylate, ethyl acrylate, *n*-propyl acrylate, isopropyl acrylate, *n*-butyl acrylate, isobutyl acrylate, *t*-butyl acrylate, *n*-pentyl acrylate, *n*-hexyl acrylate, cyclohexyl acrylate, *n*-heptyl acrylate, *n*-octyl acrylate, 2-ethylhexyl acrylate, nonyl acrylate, decyl acrylate, dodecyl acrylate, phenyl acrylate, tolyl acrylate, benzyl acrylate, isobornyl acrylate, 2-methoxyethyl acrylate, 3-methoxybutyl acrylate, 2-hydroxyethyl acrylate, 2-hydroxypropyl acrylate, stearyl acrylate, glycidyl acrylate, 2-aminoethyl acrylate, γ-(methacryloyloxypropyl)trimethoxysilane, γ-(methacryloyloxypropyl)dimethoxysilane, ethylene oxide adduct of acrylic acid, trifluoromethylmethyl acrylate, 2-trifluoromethylethyl acrylate, 2-perfluoroethylethyl acrylate, 2-perfluoroethyl-2-perfluorobutylethyl acrylate, 2-perfluoroethyl acrylate, perfluoromethyl acrylate, diperfluoromethyl methyl acrylate, 2-perfluoromethyl-2-perfluoroethyl methyl acrylate, 2-perfluorohexyl ethyl acrylate, 2-perfluorodecyl ethyl acrylate and 2-perfluorohexadecyl ethyl acrylate; aromatic alkenyl compounds such as styrene, α-methylstyrene, *p*-methylstyrene, and *p*-methoxystyrene; vinyl cyanide compounds such as acrylonitrile and methacrylonitrile; conjugated diene-base compounds such as butadiene and isoprene; halogen-containing unsaturated compounds such as vinyl chloride, vinylidene chloride, perfluoroethylene, perfluoropropylene and vinylidene fluoride; silicon-containing unsaturated compounds such as vinyl trimethoxysilane and vinyl triethoxysilane; unsaturated dicarboxylic acid compounds such as maleic anhydride, maleic acid, monoalkyl ester and dialkylester of maleic acid, fumaric acid, and monoalkyl ester and dialkyl ester of fumaric acid; vinyl ester compounds such as vinyl acetate, vinyl propionate, vinyl pivalate, vinyl benzoate and vinyl cinnamate; maleimide-base compounds such as maleimide, methyl maleimide, ethyl maleimide, propyl maleimide, butyl maleimide, hexyl maleimide, octyl maleimide, dodecyl maleimide, stearyl maleimide, phenyl maleimide and cyclohexyl maleimide; methacrylic esters such as methyl methacrylate, ethyl methacrylate, *n*-propyl methacrylate, isopropyl methacrylate, *n*-butyl methacrylate, isobutyl methacrylate, *tert*-butyl methacrylate, *n*-pentyl methacrylate, *n*-hexyl methacrylate, cyclohexyl methacrylate, *n*-heptyl methacrylate, *n*-octyl methacrylate, 2-ethylhexyl methacrylate, nonyl methacrylate, decyl methacrylate, dodecyl methacrylate, phenyl methacrylate, tolyl methacrylate, benzyl methacrylate, isobornyl methacrylate, 2-methoxyethyl methacrylate, 3-methoxybutyl methacrylate, 2-hydroxyethyl methacrylate, 2-hydroxypropyl methacrylate, stearyl methacrylate, glycidyl methacrylate, 2-aminoethyl methacrylate, γ-(methacryloyloxypropyl)trimethoxy silane, γ-(methacryloyloxypropyl)dimethoxy methyl silane, ethylene oxide adduct of methacrylic acid, trifluoromethyl methyl methacrylate, 2-trifluoromethyl ethyl methacrylate, 2-perfluoroethyl ethyl methacrylate, 2-perfluoroethyl-2-perfluorobutyl ethyl methacrylate, 2-perfluoroethyl methacrylate, perfluoromethyl methacrylate, diperfluoromethyl methyl methacrylate, 2-perfluoromethyl-2-perfluoroethyl methyl methacrylate, 2-perfluorohexyl ethyl methacrylate, 2-perfluorodecyl ethyl methacrylate, and 2-perfluorohexadecyl ethyl methacrylate; monomers derived from (meth)acrylate or (meth)acrylamide with $C_{2-4}$ alkylene oxide such as hydroxyethyl (meth)acrylate, polyethylene glycol (meth) acrylate, methoxypoly(ethylene glycol/propylene glycol)mono(meth)acrylate, polyethylene glycol di(meth)acrylate and N-polyalkyleneoxy(meth)acrylamide; and hydrophilic nonionic monomers such as N-cyclohexyl maleimide, N-phenylmaleimide, N-vinylpyrrolidone, N-(meth)acryloyl morpholine and acrylamide.

Specific Examples of Anionic Monomer:

unsaturated carboxylic acid compounds such as (meth)acrylic acid, maleic acid, maleic anhydride, itaconic acids, fumaric acid and crotonic acid; half ester compounds derived from an unsaturated polybasic acid anhydride (e. g., succinic anhydride, phthalic anhydride) and a hydroxyl-group-containing (meth)acrylate (e.g., hydroxyethyl (meth) acrylate); compounds having a sulfonic acid group such as styrenesulfonic acid and sulfoethyl (meth)acrylate; and compounds having a phosphonic acid group such as acid phosphooxyethyl(meth)acrylate.

There anionic unsaturated monomer can be used in a form of intact acid or partially-neutralized or completely-neutralized form, or can be subjected to copolymerization in a form of intact acid and can partially or completely be neutralized. Examples of the base used for the neutralization include alkali metal hydroxide such as potassium hydroxide and sodium hydroxide; aqueous ammonia solution; and amine compounds such as mono-, di- and tri-ethanolamines, and trimethylamine.

Specific Examples of Cationic Monomer:

Cationic monomers obtained by cationizing N,N-dimethylaminoethyl(meth)acrylate, N,N-diethylaminoethyl (meth)acrylate, N,N-dimethylaminopropyl (meth)acrylate, N,N-diethylaminopropyl (meth)acrylate, N,N-dimethylaminoethyl (meth)acrylamide, N,N-diethylaminoethyl (meth)acrylamide, N,N-dimethylaminopropyl (meth)acrylamide, N, N-diethylaminopropyl(meth)acrylamide, *p*-dimethylaminomethyl styrene, *p*-dimethylaminoethyl styrene, p-diethylaminomethyl styrene or p-diethylaminoethylstyrene using a cationizing agent (e.g., alkyl halides such as methyl chloride, methyl bromide and methyl iodide; dialkyl sulfates such as dimethyl sulfate; epichlorohydrin-adduct of tertiary amine mineral acid salt such as N-(3-chloro-2-hydroxypropyl)-N,N,N-trimethylammonium chloride; inorganic acids such as

hydrochloric acid, hydrobromic acid, sulfuric acid and phosphoric acid; and carboxylic acids such as formic acid, acetic acid and propionic acid).

Specific Examples of Amphoteric Monomer:

Compounds obtained by reacting the above-described examples of the cationic monomer precursors with a modifying agent such as sodium haloacetate or potassium.

Specific Examples of Polarized Monomer:

Amine oxides of N, N-dimethylaminoethyl (meth)acrylate, N,N-diethylaminoethyl (meth)acrylate, N,N-dimethylaminopropyl (meth)acrylate, N,N-diethylaminopropyl (meth)acrylate, N,N-dimethylaminoethyl (meth)acrylamide, N, N-diethylaminoethyl (meth)acrylamide, N,N-dimethylaminopropyl (meth)acrylamide, N,N-diethylaminopropyl(meth) acrylamide, vinyl N,N-dimethylaminopropionate, *p*-dimethylaminomethylstyrene, *p*-dimethylaminoethylstyrene, *p*-diethylaminomethylstyrene and *p*-diethylaminoethylstyrene.

These monomers can be used in a singular manner or in any combination of two or more species. Among these, acrylic esters, methacrylic esters, aromatic alkenyl compounds, vinyl cyanide compounds, conjugate diene-base compounds or halogen-containing unsaturated compounds are preferably used in view of its readiness in industrial availability.

A preferable embodiment of the block copolymer can be exemplified by a block copolymer comprising at least one species each of a block composed of an ethylenic unsaturated carboxylic acid unit, and a block composed of an ethyleninc unsaturated carboxylate ester unit. The ethylenic unsaturated carboxylic acid unit is preferably a unit which is derived from a hydrophilic monomer and has a high Tg. Preferred examples of such a unit include units derived from acrylic acid and methacrylic acid. On the other hand, the ethylenic unsaturated carboxylate ester unit is preferably a unit which is derived from a hydrophobic monomer and has a low Tg. Preferred examples of such a unit include units derived from acrylic esters and methacrylic esters. Compositional ratios of the ethylenic unsaturated carboxylic acid block and the ethylenic unsaturated carboxylate ester block composing the polymer are preferably 10 to 90% by weight for the former, and 90 to 10% by weight for the latter, more preferably 15 to 80% by weight for the former, and 80 to 15% by weight for the latter, and still more preferably 20 to 50% by weight for the former, and 80 to 50% by weight for the latter. The ethylenic unsaturated carboxylic acid block less than 10% by weight tends to make the block copolymer insoluble into water, and the ethylenic unsaturated carboxylate ester block less than 10% by weight tends to worsen the film-forming ability and to considerably degrade rubber elasticity of the polymer.

It is to be noted that "unit derived from a compound" in the context of this patent specification means not only a unit obtained after polymerizing the compound as a monomer, but also means a unit structurally derived from the compound obtained after the post-treatment such as hydrolysis as described in the above.

The block copolymer may have any form of diblock copolymer, triblock copolymer and multi-block copolymer, without departing from the straight-chain structure. For example, the block copolymer having a hard block A (high-Tg block) and a soft block B (low-Tg block) may have any form of A-B diblock copolymer, A-B-A triblock copolymer, B-A-B triblock copolymer and $(A-B)_n$ multi-block copolymer. Among theses, A-B-A triblock copolymer, $(A-B)_n$ multi-block copolymer and mixture of these are preferable in view of providing rubber elasticity to the polymer.

The block copolymer has a straight-chain structure.

The block copolymer is preferably dispersible or soluble in water and/or alcohol. The solubility in water (or solubility in alcohol) of the block copolymer can be confirmed by stirring 1 weight part of the block copolymer and 99 parts by weight of deionized water and/or ethanol-mixed solution at 60°C for 2 hours, cooling the mixture, allowing the mixture to stand at room temperature for a day, and observing that the resultant aqueous solution is kept homogeneous without producing precipitate, and shows a light transmissivity of 70% or more at 655 nm. "Dispersible" in the context of this patent specification means that fine particles of the copolymer can disperse in water and/or alcohol without causing precipitation, and is kept in an emulsified form or latex form.

The block copolymer is preferably manufactured by controlled polymerization. The controlled polymerization can be exemplified by living anionic polymerization, radical polymerization using a chain-transfer agent, and recently-developed living radical polymerization. Among these, living radical polymerization, a sort of controlled radical polymerization, is preferable because the method is successful in readily controlling the molecular weight and structure of the resultant block copolymer.

Living radical polymerization refers to a radical polymerization during which activity of the polymerization terminal is kept without being lost. Living polymerization in a narrow sense refers to polymerization during which the terminal keeps on maintaining the activity, but is generally understood as including also pseudo-living polymerization in which inactivated terminal and activated terminal are kept in an equilibrium. The latter meaning is adopted in this patent specification. In recent years, living radical polymerization has extensively been studied by many teams, and

known achievements include a method using a chain-transfer agent such as polysulfide; methods using radical trapping agents such as cobalt porphyrin complex (J. m. Chem. Soc., 1994, 116, 7943) and nitroxide compound (Macromolecules, 1994, 27, 7228); and atom-transfer radial polymerization (ATRP) using an organic halide as an initiator, and using a transition metal complex as a catalyst. Any of these methods may be adopted to the present invention, where atom-transfer radical polymerization is preferably adopted in view of simplicity in the control.

In atom-transfer radical polymerization, organic halide or halogenated sulfonyl compound can be used as an initiator, and a metal complex, having a metal selected from Group VIII, Group IX, Group X and Group XI elements in the periodic table as a central metal, can be used as a catalyst. Specific processes of the atom-transfer radical polymerization, typically described by Matyjaszewski *et al*. (J. Am. Chem. Soc. 1995, 117, 5614; Macromolecules, 1995, 28, 7901 and Science, 1996, 272, 866) and by Sawamoto *et al.* (Macromolecules, 1995, 28, 1721), are preferably used also for the present invention. These methods can successfully proceed in a living manner despite that the polymerization speed is extremely large and the radical polymerization system is highly causative of termination reaction typically due to inter-radical coupling, and can produce a polymer having a narrow molecular weight distribution, namely has a Mw/Mn of 1.1 to 1.5 or around. The molecular weight can freely be controlled by charged amount ratio of the monomer to initiator.

In the atom-transfer radical polymerization, mono-functional, bi-functional or poly-functional organic halide, or halogenated sulfonyl compound can be used as an initiator. These may appropriately be selected depending on purposes, where mono-functional compound is preferably used as an initiator for the manufacture of the diblock copolymer, and bi-functional compound is preferably used for the manufacture of the triblock copolymer.

Examples of the mono-functional compounds which can be used as an initiator are listed below:

$C_6H_5$-$CH_2X$;
$C_6H_5$-C(H) (X)-$CH_3$;
$C_6H_5$-C(X) $(CH_3)_2$;
$R^{11}$-C(H) (X)-$COOR^{12}$;
$R^{11}$-C($CH_3$) (X)-$COOR^{12}$;
$R^{11}$-C(H) (X)-CO-$R^{12}$;
$R^{11}$-C($CH_3$) (X)-CO-$R^{12}$; and
$R^{11}$-$C_6H_4$-$SO_2X$.

In these formulae, $C_6H_4$ represents a phenylene group (any of ortho-substitute, meta-substitute and para-substitute allowable). $R^{11}$ represents a hydrogen atom, $C_{1-20}$alkyl group, $C_{6-20}$ aryl group or $C_{7-20}$ aralkyl group. X represents chlorine, bromine or iodine. $R^{12}$ represents a $C_{1-20}$ mono-valent organic group.

Examples of the bi-functional compounds which can be used as an initiator are listed below:

X-$CH_2$-$C_6H_4$-$CH_2$-X;
X-CH($CH_3$)-$C_6H_4$-CH($CH_3$)-X;
X-C$(CH_3)_2$-$C_6H_4$-C$(CH_3)_2$-X;
X-CH($COOR^{13}$)-$(CH_2)_n$-CH($COOR^{13}$)-X;
X-C($CH_3$)($COOR^{13}$)-$(CH_2)_n$-C($CH_3$)($COOR^{13}$)-X;
X-CH($COR^{13}$)-$(CH_2)_n$-CH($COR^{13}$)-X;
X-C($CH_3$)($COR^{13}$)-$(CH_2)_n$-C($CH_3$)($COR^{13}$)-X;
X-$CH_2$-CO-$CH_2$-X;
X-CH($CH_3$)-CO-CH($CH_3$)-X;
X-C$(CH_3)_2$-CO-C$(CH_3)_2$-X;
X-CH($C_6H_5$)-CO-CH($C_6H_5$)-X;
X-$CH_2$-COO-$(CH_2)_n$-OCO-$CH_2$-X;
X-CH($CH_3$)-COO-$(CH_2)_n$-OCO-CH($CH_3$)-X;
X-C$(CH_3)_2$-COO-$(CH_2)_n$-OCO-C$(CH_3)_2$-X;
X-$CH_2$-CO-CO-$CH_2$-X;
X-CH($CH_3$)-CO-CO-CH($CH_3$)-X;
X-C$(CH_3)_2$-CO-CO-C$(CH_3)_2$-X;
X-$CH_2$-COO-$C_6H_4$-OCO-$CH_2$-X;
X-CH($CH_3$)-COO-$C_6H_4$-OCO-CH($CH_3$)-X;
X-C$(CH_3)_2$-COO-$C_6H_4$-OCO-C$(CH_3)_2$-X; and
X-$SO_2$-$C_6H_4$-$SO_2$-X.

In these formulae, $R^{13}$ represents a $C_{1-20}$ alkyl group, $C_{6-20}$ aryl group or $C_{7-20}$ aralkyl group, where two or

more $R^{13}$ groups exist in one molecule may be same or different each other. $C_6H_4$ represents a phenylene group (any of ortho-substitute, meta-substitute and para-substitute allowable). $C_6H_5$ represents a phenyl group, n is an integer from 0 to 20, and X represents chlorine, bromine or iodine.

Examples of the poly-functional compounds which can be used as an initiator are listed below:

$C_6H_3$-($CH_2$-X)$_3$;
$C_6H_3$-(CH($CH_3$)-X)$_3$;
$C_6H_3$-(C($CH_3$)$_2$-X)$_3$;
$C_6H_3$-(OCO-$CH_2$-X)$_3$;
$C_6H_3$-(OCO-CH($CH_3$)-X)$_3$;
$C_6H_3$-(OCO-C ($CH_3$)$_2$-X)$_3$; and
$C_6H_3$-($SO_2$-X)$_3$.

In these formulae, $C_6H_3$ represents a three-substituted phenyl group (any position of substituents on the 1- to 6-positions allowable). X represents chlorine, bromine or iodine.

Use of organic halide or halogenated sulfonyl compound having a functional group not contributable to initiation of the polymerization is successful in readily obtaining a polymer having such functional group on the molecular terminal. Examples of the functional group can be exemplified by alkenyl group, hydroxyl group, epoxy group, amino group, amido group and silyl group.

The organic halide and halogenated sulfonyl compound which can be used as an initiator are such as those having an activated carbon-halogen bond, the carbon of which is also bound to a carbonyl group, phenyl group or the like, thereby exhibiting the polymerization initiation property.

The amount of use of the initiator may be determined in terms of ratio to the monomer, considering the molecular weight required for the block copolymer. In other words, the molecular weight of the block copolymer can be controlled by the number of monomer molecules used per one initiator molecule.

The transition metal catalyst used as a catalyst in the atom-transfer radical polymerization is not specifically limited, where preferable examples include monovalent and zero-valent copper, divalent ruthenium, divalent iron and divalent nickel complexes. Among these, copper complex is preferably used in view of cost and controllability of the reaction. When the complex is used as the metal catalyst, the complex may preliminarily be synthesized and added to the polymerization system, or may be produced in the system by adding a metal salt and ligand capable of forming a complex with the metal through coordinate bond respectively into the polymerization system. Among these, a preferable method is such as using copper halide as the metal catalyst together with an amine compound capable of forming coordinate bond with copper, and adding these substances into the polymerization system.

Monovalent copper compounds which can be used as a catalyst for the atom-transfer radical polymerization can be exemplified typically by copper (I) chloride, copper (I) bromide, copper (I) iodide, copper (I) cyanide, copper (I) oxide and copper (I) perchlorate. The copper compound can preferably be used together with a ligand for enhancing the catalytic activity, and examples of the ligand include 2,2'-bipyridyl and its derivatives, 1,10-phenanthroline and its derivatives, and polyamines such as tetramethyletylene triamine(TMEDA), pentamethyldiethylene triamine and hexamethyl(2-aminoethyl)amine. Divalent tris-triphenylphosphine complex of ruthenium ($RuCl_2(PPh_3)_3$) is also preferable as the catalyst. Use of ruthenium as the catalyst may preferably be associated with addition of aluminum alkoxide as an activator. Also bis-triphenylphosphine complex of divalent iron ($FeCl_2(PPh_3)_2$), bis-triphenylphosphine complex of divalent nickel ($NiCl_2(PPh_3)_2$), and bis-tributylphosphine complex of divalent nickel ($NiBr_2(PBu_3)_2$) are good catalysts. There are no specific limitations on the catalyst to be used, and amount of use of the ligand and activator, and may appropriately be determined considering relations with the amounts of initiator, monomer and solvent, and a desired reaction speed.

The atom-transfer radical polymerization can be proceeded under a solventless condition (bulk polymerization) or in a variety of solvents. Examples of available solvents include hydrocarbon solvents such as benzene and toluene; ether solvents such as diethyl ether and tetrahydrofuran; halogenated hydrocarbon solvents such as methylene chloride and chloroform; ketone solvents such as acetone, methyl ethyl ketone and methyl isobutyl ketone; alcohol solvents such as methanol, ethanol, propanol, isopropanol, *n*-butanol and *t*-butanol; nitrile solvents such as acetonitrile, propionitrile and benzonitrile; ester solvents such as ethyl acetate and butyl acetate; carbonate solvents such as ethylene carbonate and propylene carbonate; and other solvents such as dimethylformamide, dimethylsulfoxide, N-methylpyrrolidone and water. These solvents may be used in a singular manner or as a mixture of two or more species. The solventless process is referred to as bulk polymerization as described in the above. On the other hand, when any solvent is used, the amount of addition of the solvent may appropriately be determined considering relation between viscosity of the entire system and necessary stirring efficiency (i.e., reaction speed).

The atom-transfer radical polymerization can be proceeded within a range from room temperature to 200°C, and more preferably from 50 to 150°C.

Specific methods for manufacturing the block copolymer based on the atom-transfer radial polymerization include a method in which the monomer is sequentially added; a method in which a preliminarily-synthesized polymer is used as a polymer initiator for polymerizing the next block; and a method in which separately-prepared monomers are allowed to bind through reaction. These methods may properly be selected depending on purposes, where the method based on the sequential addition of the monomer or use of the polymer initiator are preferable in view of simplicity of the manufacturing process.

There is no specific limitation on the method of manufacturing the block copolymer, where the controlled radical polymerization based on the sequential addition of the monomers using copper halide as a catalyst together with amine ligand is preferable since such method is successful in facilitating control of the molecular weight of the resultant block copolymer. In particular, use of dimethylformamide as a reaction solvent is more preferable because the reaction speed can be increased based on chelate effect with respect to the catalyst.

The block copolymer obtained by the polymerization may be used for the cosmetic polymer composition of the present invention in an intact form, or used after being subjected to the post-treatment such as hydrolysis. Control of the modification ratio by the post-treatment is successful in adjusting various characteristics of the resultant block copolymer, such as water solubility and film-forming ability, within a desirable range adaptive to the applications. Examples of the post-treatment include hydrolysis, quaternization and amine-oxide-forming treatment. An exemplary hydrolytic process can hydrolyze a block formed of acrylic ester, methacrylic ester or the like, to thereby produce an acrylic acid-derived or methacrylic acid-derived block having carboxylic acid group as a hydrophilic group (e.g., a block having he unit represented by the formula (1)). Hydrolysis of ester can be carried out by using an acid catalyst such as hydrochloric acid and p-toluenesulfonic acid, or a base catalyst such as sodium hydroxide. The degree of hydrolysis can be controlled by the amount of use of the catalyst and reaction time. The carboxylic acid thus produced by the hydrolysis may be used after being partially or completely neutralized. A substance conveniently used for the neutralization is base, and examples of the base include alkali metal hydroxide such as potassium hydroxide and sodium hydroxide: and amine compounds such as aqueous ammonia solution, mono-, di- and tri-ethanolamines and trimethylamine.

Quaternization of a block composed of amino-group-containing methacrylate or the like can produce a block having a cationic quaternary ammonium group (e.g., the block represented by the formula (2)). The quaternization can be proceeded by using a cationizing agent, examples of which include alkyl halides such as methyl chloride, methyl bromide and methyl iodide; dialkyl sulfates such as dimethyl sulfate; epichlorohydrin-adduct of tertiary amine mineral acid salt such as N-(3-chloro-2-hydroxypropyl)-N,N,N-trimethylammonium chloride; inorganic acids such as hydrochloric acid, hydrobromic acid, sulfuric acid and phosphoric acid; and carboxylic acids such as formic acid, acetic acid and propionic acid). It is also possible to form a block having amine oxide group, which is a polarized group, by subjecting a block composed of amino-group-containing methacrylate or the like to amine-oxide-forming treatment (e.g., the block represented by the formula (3)). The amine-oxide-forming treatment can be carried out by using peracids such as hydrogen peroxide, ammonium persulfate, sodium persulfate, peracetic acid, meta-chloroperbenzoic acid, benzoyl peroxide, *t*-butylhydro peroxide and so forth; and an oxidizing agent such as ozone:

The copolymer used for the cosmetic polymer composition of the present invention is preferably capable of forming a film having a Young's modulus of 50 MPa to 1,000 MPa, and an elongation percentage of 100% to 1,000%. Young's modulus less than 50 MPa may reduce the effect of the present invention, and on the other hand, exceeding 1,000 MPa may excessively harden the film to thereby degrade the feel of touch. The elongation percentage of the film less than 100% may reduce the effect of the present invention, and an effort to obtain a flexibility as large as exceeding 1,000% tends to excessively increase the production cost typically because a specialized monomer is necessary. The copolymer is more preferably capable of forming a film having a Young's modulus of 100 Mpa to 1, 000 Mpa, and an elongation percentage of 150% to 1000%. Young's modulus and elongation percentage can be measured by a film tensile test conforming to JIS K7161. If the block copolymer can form a film having Young's modulus and elongation percentage within the above-described ranges, the cosmetic polymer composition of the present invention applied to a hair cosmetic material is successful in ensuring a desirable styling ability, and in providing a hair cosmetic material excellent in the feel of touch.

II. (b1) Anionic Polymer

One embodiment of the cosmetic polymer composition of the present invention relates to a composition containing the block copolymer as component (a), and an anionic polymer having an anionic group as component (b1). This embodiment is preferable as a hair cosmetic polymer composition. The anionic polymer is preferably selected from the group consisting of polymers having a carboxyl group, s sulfonic acid group, a phosphonic acid group and salts of these groups. Specific examples of the anionic polymer available as the component (b1) include methyl vinyl ether/maleic anhydride alkyl half ester copolymer such as Gantrez ES-225, ES-425, A-425, V-225, V-425 (all of these are products of ISP) ; vinyl acetate/crotonic acid copolymer such as Resin 28-1310 (product of National Starch and

Chemical Company) and Luviset CA (product of BASF AG);vinyl acetate/crotonic acid/vinyl neodecanoate copolymer such as Resin 28-2930 (product of National Starch and Chemical Company); vinyl acetate/crotonic acid/vinyl propionate copolymer such as Luviset CAP (product of BASF AG); vinyl acetate/monobutyl maleate/isobornyl acrylate copolymer such as Advantage CP (product of ISP); (meth)acrylic acid/(meth)acrylate copolymer such as Luvimer 100P (product of BASF AG) and Diahold (product of Mitsubishi Chemical Corporation); acrylic acid/acrylamide derivative copolymer such as Ultrahold=Strong, Ultrahold 8 (two theses products are from BASF AG), Versatyle 42 (product of National Starch and Chemical Company) and Plascize L53P (product of Goo Chemical Co., Ltd.); poly(vinylpyrrolidone/(meth)acrylic acid/(meth)acrylate copolymer such as Luviflex VBM35 (product of BASF AG); diethylene glycol/cyclohexane dimethanol/dimethyl isophthalate/dimethyl sulfonated isophthalate condensate such as Eastman AQ Polymer (product of Eastman Chemical Company).

The anionic group in the anionic polymer (b1) is preferably used in a form of partially or completely neutralized with a basic compound, in view of water solubility. Examples of such basic compound include alkali metal hydroxide such as sodium hydroxide and potassium hydroxide; inorganic basic compounds such as aqueous ammonia; alkanol amines such as ethanolamine, diethanolamine, triethanolamine, triisopropanolamine, 2-amino-2-methyl-1-propanol, 2-amino-2-methyl-1,3-propanediol, aminomercaptopropanediol; basic amino acids such as lysine, arginine and histidine. Among these, 2-amino-2-methyl-1-propanol and potassium hydroxide are particularly preferable in view of water solubility.

The anionic polymer (b1) preferably contain the anionic group in an amount of 5% by weight or more, more preferably 10% by weight or more, and still more preferably 15% by weight or more.

Weight-average molecular weight of the anionic polymer (b1) preferably falls within a range from 5, 000 to 1, 000, 000, more preferably from 10,000 to 500,000, and still more preferably from 20,000 to 300,000.

In one embodiment relating to the hair cosmetic polymer composition, ratio by weight ((a)/(b1)) of the block copolymer (a) to anionic polymer (b1) preferably falls in a range from 1/10 to 10/1, and more preferably from 1/5 to 10/1. The ratio (a)/(b1) less than 1/10 may result in a poor flexibility, a stiff touch on the hair, a poor styling ability, and difficulty in setting hair due to flaking or static electricity during combing hairs. On the other hand, the ratio exceeding 10/1 may result in a poor elasticity and volume in the hair styling due to insufficient hardness, a heavy finish, and a degraded feel of touch in the finished style. The hair cosmetic polymer composition of the present invention preferably contains the component (a) and component (b1) in an amount of 0.1 to 10% by weight of the total composition, and more preferably 0.5 to 8% by weight. A content of less than 0.1% by weight tends to result in a poor hair styling ability, and of exceeding 10% by weight may add a stiff touch and worsen the feel of touch.

III. (b2) Cationic Polymer

Another embodiment of the present invention relates to a composition containing the block copolymer as component (a), and a cationic polymer having a cationic group as component (b2). This embodiment is preferable as a hair cosmetic polymer composition. The cationic polymers which can be used as the component (b2) are such as those listed as ① to ④ below:

① copolymer of which constituents are N-vinylpyrrolidone and/or N-vinylcaprolactam and a cationic-group-containing monomer;
② polymer or copolymer of dimethyl diallyl ammonium;
③ polymer or copolymer of acrylic-ester-base or methacrylic-ester-base quaternary ammonium salt; and
④ quaternary ammonium salt of cellulose-base or chitosan-base polymer.

As the cationic polymer (b2), synthetic cationic polymers listed as ① to ③ , and cationic polymers obtained by modifying the natural products listed as ④ can be used. It is to be noted that the examples described hereinafter contains, as the "cationic polymer", a polymer cationizable by the post-treatment such as neutralization, but any form of cationization is allowable for the polymer.

Examples of the synthetic cationic polymer include N-vinylpyrrolicone/dimethylaminoethyl methacrylate copolymer such as Gafquat 755N, 755, 734 (all of these are products of ISP) and Luviquat PQ11 (product of BASF AG); N-vinylpyrrolidone/dimethylaminoethyl methacrylate copolymer such as Copolymer 845, 937 and 958 (products of ISP) ; N-vinylpyrrolidone/N-vinylcaprolactam/dimethylaminoethyl methacrylate copolymer such as Gaffix VC-713 (product of ISP); N-vinylpyrrolidone/methacrylamidopropyl trimethylammonium chloride copolymer such as Gafquat HS-100 (product of ISP); N-vinylpyrrolidone/quaternized methyl vinyl imidazolium copolymer such as Luviquat FC370, FC550, FC905, HM-552 (these are products of BASF AG); dimethyldiallylammonium chloride polymer such as Marcoat 100, 550 (products of Calgon); dimethyldiallylammonium chloride/acrylamide copolymer; and quaternerized dialkylaminoalkylene methacrylate/(meth)acrylate alkyl ester copolymer such as those disclosed in Japanese Laid-Open Patent Publication Nos. hei 4-21623 and hei 5-310538.

Examples of the modified-natural-product-base cationic polymer include hydroxyethyl cellulose/dimethyldiallylammonium chloride copolymer such as Celquat H-100, L200 (products of National Starch and Chemical Company); reaction product of hydroxyethyl cellulose with epoxized trimethylammonium compound such as Celquat SC-240, SC-240C, SC-230M (products of National Starch and Chemical Company), Ucare Polymer JR-125, JR-400, JR-30M (products of Amacol), Leoguard G (product of Lion Corporation), Catinal HC, LC (products of Toho Chemical Industry Co., Ltd.); and quaternized Kitamer KC (product of Amacol).

The cationic polymer as the component (b2) preferably contains the unit composed of the cationic polymer or its quaternized product in an amount of 5% by weight or above, more preferably 10% by weight or above, and still more preferably 15% by weight or above.

The weight-average molecular weight of the cationic polymer (b2) is preferably 5,000 to 1,000,000, more preferably 10,000 to 500,000, and still more preferably 20,000 to 300,000.

In this embodiment relating to the hair cosmetic polymer composition, ratio by weight ((a)/(b2)) of the block copolymer (a) and the cationic polymer (b2) is preferably falls in a range from 1/10 to 10/1, and more preferably from 1/5 to 10/1. The ratio of (a)/(b2) less than 1/10 may fail in keeping a desired hair style due to a poor styling effect, and may result in sticking touch due to shortage of elasticity of the hair under high humidity. In addition, a long-term repetitive use may raise a problem of build-up. The ratio exceeding 10/1 may result in a poor slipping nature when applied to the hair, and this makes combing difficult, and reduces the smoothness after being dried.

This embodiment relating to a cosmetic polymer composition preferably contains the component (a) and component (b2) in a total amount of 0.1 to 10% by weight of the entire composition, and more preferably 0. 5 to 8% by weight. The total amount of less than 0.1% by weight tends to result in only an insufficient styling power, and exceeding 10% by weight may increase the stiff touch and therefore worsen the feel of touch.

IV. (b3) Nonionic Polymer

Another embodiment of the present invention relates to a composition containing, together with the block copolymer as component (a), a nonionic polymer having a nonionic group as component (b3). This embodiment is preferable as a hair cosmetic polymer composition. The nonionic polymers which can be used in this embodiment as the component (b3) are preferably selected from the polymers whose constituent is an unsaturated monomer having at least one functional group selected from the group consisting of pyrrolidone group, amido group (including N-alkyl amido), polyether group, formamide group and acetamide group.

Specific examples of the polymer having pyrrolidone group include polyvinylpyrrolidone such as Luviskol K-12, K-17, K-30, K-60, K-80, K-90 (all of these are products of BASF AG), PVP K-15, K-30, K-60, K-90 and K-120 (products of ISP); vinylpyrrolidone/vinyl acetate copolymer such as Luviskol VA28, VA37, VA55, VA64, VA73 (products of BASF AG), PVP/VA-735, PVP/VA-635, PVP/VA-535, PVP/VA-335, PVP/VA-235 and S-630 (products of ISP); and vinylpyrrolidone/vinyl acetate/vinyl propionate copolymer such as Luviskol VAP343 (product of BASF AG).

Examples of the polymer whose constituent is an unsaturated monomer having an amido group, N-alkyl-substituted amido group or polyether group include homopolymers of an unsaturated monomer such as (meth)acrylamide, N-octyl (meth)acrylamide, hydroxyethyl (meth)acrylate, methoxypolyethylene glycol (meth)acrylate, and methoxypolyethylene glycol · polypropylene glycol (meth)acrylate; or copolymers with (meth)acrylate alkyl ($C_{1-24}$) ester, vinyl acetate or the like.

Examples of the polymer whose constituent is an unsaturated monomer containing an acetamide group or formamide group include homopolymers of an unsaturated monomer such as N-vinylacetamide and N-vinylformamide, and radical copolymer with alkyl (meth)acrylate ($C_{1-24}$) ester or vinyl acetate.

The nonionic polymer as the component (b3) preferably contains the nonionic group in an amount of 10% by weight or above, more preferably 20% by weight or above, and still more preferably 30% by weight or above.

The weight-average molecular weight of the nonionic polymer (b3) is preferably 5,000 to 1,000,000, more preferably 10,000 to 500,000, and still more preferably 20,000 to 300,000.

In this embodiment relating to a cosmetic polymer composition, ratio by weight ((a)/(b3)) of the block copolymer (a) and the nonionic polymer (b3) is preferably falls in a range from 1/10 to 10/1, and more preferably from 1/5 to 10/1. The ratio of (a)/(b3) less than 1/10 may result in a poor flexibility, a stiff touch on the hair, a poor styling effect, and poor setting property of the hair by combing due to flaking or static electricity. On the other hand, the ratio exceeding 10/1 may result in a poor elasticity and volume in the hair styling due to insufficient hardness, a heavy finish, and a degraded feel of touch in the finished style.

This embodiment relating to a cosmetic polymer composition preferably contains the component (a) and component (b3) in a total amount of 0.1 to 10% by weight of the entire composition, and more preferably 0.5 to 8% by weight. The total amount of less than 0.1% by weight tends to result in only an insufficient styling power, and exceeding 10% by weight may increase the stiff touch and therefore worsen the feel of touch.

V. Amphoteric Polymer

Another embodiment of the present invention relates to a composition containing, together with the block copolymer as component (a), an amphoteric polymer having an amphoteric group as component (b4). This embodiment is preferable as a hair cosmetic polymer composition. The amphoteric polymer which can be used as the component (b4) is preferably a polymer whose constituent is a monomer having a betaine-structured group such as carboxybetaine group, sulfobetaine group or phosphobetaine group; or a polymer whose constituents are both of an unsaturated monomer having an anionic group such as carboxyl group, sulfonic acid group or phosphonic acid group, and an unsaturated monomer having a quaternary ammonium salt group.

Examples of the polymer whose constituent is an unsaturated monomer having a betaine-structured group include methacryl-base carboxybetaine polymer which is a monohaloacetate-modified product of dimethylaminoethyl methacrylate/alkyl methacrylate copolymer, such as Yukaformer 205S, SM, AMPHOSET 301, R102, R402, 510, 201 and W (products of Mitsubishi Chemical Corporation), which are disclosed in Japanese Laid-Open Patent Publication Nos. syo 51-9732, syo 55-104209, syo 61-258804, and hei 7-285832.

Examples of the polymer whose constituents are both of an unsaturated monomer having an anionic group such as carboxyl group, sulfonic acid group or phosphonic acid group, and an unsaturated monomer having a quaternary ammonium salt group include a quaternized product of a polymer whose constituents are both of an unsaturated monomer having a tertiary amino group and an unsaturated monomer having a carboxyl group, which is typified by hydroxypropyl/buthylaminoethyl methacrylate/octylamide acrylate/acrylate copolymer, such as Amphomer 28-4910, LV-71 and LV-47 (products of National Starch and Chemical Company); and a polymer whose constituents are both of a carboxyl-group-containing unsaturated monomer and a quaternary-ammonium-salt-group-containing unsaturated monomer, such as Marcoat 295 (product of Cargon), which is diallyl dimethyl ammonium chloride/acrylic acid copolymer, and as MarcoatPlus 3330 (product of Cargon), which is diallyl dimethyl ammonium chloride/acrylic acid/acrylamide copolymer.

The amphoteric polymer as the component (b4) preferably contains the amphoteric group in an amount of 5% by weight or above, more preferably 10% by weight or above, and still more preferably 15% by weight or above.

The weight-average molecular weight of the ampoteric polymer (b4) is preferably 5,000 to 1,000,000, more preferably 10,000 to 500,000, and still more preferably 20,000 to 300,000.

In this embodiment relating to a cosmetic polymer composition, ratio by weight ((a)/(b4)) of the block copolymer (a) and the amphoteric polymer (b4) is preferably falls in a range from 1/10 to 10/1, and more preferably from 1/5 to 10/1. The ratio of (a)/(b4) less than 1/10 may fail in keeping a desired hair style due to a poor styling effect, and may result in a reduced elasticity of the hair under high humidity. On the other hand, the ratio exceeding 10/1 may result in a poor slipping nature when applied to the hair, and this makes combing difficult, and produces a feel of creaking.

This embodiment relating to a cosmetic polymer composition preferably contains the component (a) and component (b4) in a total amount of 0.1 to 10% by weight of the entire composition, and more preferably 0. 5 to 8% by weight. The total amount of less than 0.1% by weight tends to result in only an insufficient styling power, and exceeding 10% by weight may increase the stiff touch and therefore worsen the feel of touch.

VI. Amine Oxide Group-Containing Polymer

Another embodiment of the present invention relates to a composition containing, together with the block copolymer (a), an amine-oxide-group-containing polymer (b5). This embodiment is preferable as a hair cosmetic polymer composition. The amine-oxide-group-containing polymer (b5) preferably has a polymer structure composed of an amine-oxide-group-containing unsaturated monomer (i), and more preferably has a copolymer structure composed of (i) and a hydrophobic monomer (ii). In this type of copolymer, compositional ratio of the amine-oxide-group-containing monomer is preferably 15 to 90% by weight, and compositional ratio of the hydrophobic monomer is preferably 85 to 10% by weight. The compositional ratio of (i) of less than 15% by weight lowers the water solubility of the resultant copolymer, and tends to make it difficult to remove the copolymer by hair wash, and on the other hand, the compositional ratio exceeding 90% by weight may cause a tacky feel of touch.

The hydrophobic monomer (ii) is preferably selected from ethylenic unsaturated carboxylic acid esters.

The weight-average molecular weight of the amine-oxide-group-containing polymer (b5) is preferably 5,000 to 1,000,000, more preferably 10,000 to 500,000, and still more preferably 20,000 to 300,000.

The amine-oxide-containing polymer (b5) can be manufactured by any one of methods selected from ① to ④ below. Among these, method ② is preferable. It is to be noted that, in this specification, any products obtained by any of these methods are generally referred to as "having a polymer structure composed of amine-oxide-group-containing unsaturated monomer".

① A method of polymerizing an amine-oxide-group-containing monomer (i) obtained by oxide-forming reaction

of a nitrogen-containing monomer;
② a method of polymerizing a nitrogen-containing monomer and then subjecting the nitrogen-containing group to oxide-forming reaction;
③ a method of polymerizing a monomer having a reactive functional group, and then allowing the product to react with a compound having both of an active group, reactive to the functional group, and an amine oxide group; and
④ a method of polymerizing a monomer having a reactive functional group, allowing the product to react with a compound having both of an active group, reactive to the functional group, and a nitrogen-containing group, and then subjecting the nitrogen-containing group to oxide-forming reaction.

Specific examples of the amine-oxide-containing polymer (b5) include methacryl-base, amine-oxide-group-containing polymer, which is an amine-oxide-modified product of dimethylaminoethyl methacrylate/alkyl methacrylate copolymer, such as Diaformer Z-711, Z-712 and Z-731 (products of Mitsubishi Chemical Corporation) (see Japanese Laid-Open Patent Publication No. 10-72323).

As described in the above, the amine-oxide-group-containing polymer preferably has a polymer structure formed of amine-oxide-group-containing unsaturated monomer (i), or more preferably has a copolymer structure formed of amine-oxide-group-containing unsaturated monomer (i) and a hydrophobic monomer (ii) or preferably an ethylenic unsaturated carboxylate ester.

Specific examples of the amine-oxide-group-containing monomer (i) include those represented by the formulae (b5-1) to (b5-4).

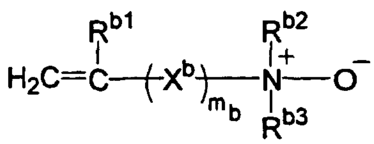
(b5-1)

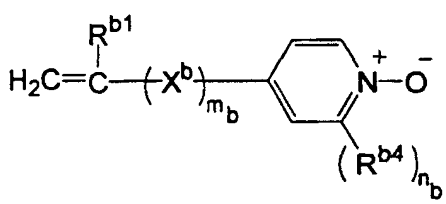
(b5-2)

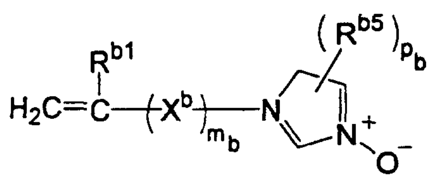
(b5-3)

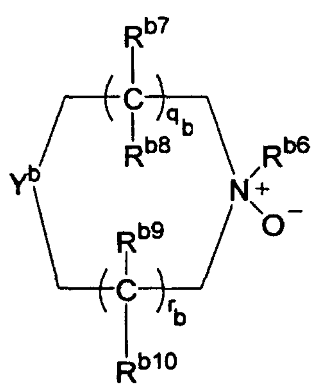
(b5-4)

where, $R^{b1}$ represents a hydrogen atom or methyl group, $R^{b2}$ and $R^{b3}$ represent a $C_{1-24}$ alkyl group, aryl group or $C_{7-24}$ aralkyl group, which may be identical or may differ from each other; $R^{b4}$ and $R^{b5}$ independently represent $C_{1-24}$ alkyl group, $C_{6-24}$ aryl group or aralkyl group; $X^b$ represents a divalent bonding group; $m_b$ is an integer of 0 or 1; $n_b$ is an integer from 0 to 4; $p_b$ is an integer from 0 to 3; $Y^b$ represents at least one divalent bonding group selected from the group consisting of -C($R^{b11}$) ($R^{b12}$)-, -N($R^{b13}$)-, -S- and -O-. At least one of $R^{b6}$ to $R^{b13}$ represents a double-bond-containing groups represented by $CH_2$=C($R^{b1}$)-($X^b$)$_{mb}$-, and other $R^{b6}$ to $R^{b13}$ respectively represent a hydrogen atom, $C_{1-24}$ alkyl group, $C_{6-24}$ aryl group or aralkyl group; and $q_b$ and $r_b$ independently represent an integer from 1 to 10.

Specific examples of the monomer represented by the formula (b5-1) include amine oxide compounds such as N,N-dimethylaminoethyl acrylate, N,N-dimethylaminoethyl methacrylate (abbreviated as N,N-dimethylaminoethyl (meth)acrylate, hereinafter), N,N-diethylaminoethyl (meth)acrylate, N,N-dimethylaminopropyl (meth)acrylate, N,N-diethylaminopropyl (meth)acrylate, N,N-dimethylaminoethyl (meth)acrylamide, N,N-diethylaminoethyl (meth)acrylamide, N,N-dimethylaminopropyl (meth)acrylamide, N,N-diethylaminopropyl (meth)acrylamide, vinyl N,N-dimethylaminopropionate, *p*-dimethylaminomethylstyrene, *p*-dimethylaminoethylstyrene, *p*-diethylaminomethylstyrene and *p*-diethylaminoethylstyrene; reaction products obtained by reacting an unsaturated-group-containing acid anhydride such as maleic anhydride, itaconic anhydride and crotonic anhydride with a compound having both of a group reactive to these acid anhydride group and a tertiary amino group such as N,N-dimethyl-1,3-propaneamine and N,N-dimethyl-*p*-phenylenediamine; and reaction products obtained by reacting an epoxy-group-containing monomer such as glycidyl methacrylate with a compound having both of a group reactive to these epoxy groups and a tertiary amino group such as N,N-dimethyl-1,3-propaneamine, N,N-dimethyl-*p*-phenylenediamine. Other examples include reaction products obtained by reacting an epoxy-group-containing monomer such as glycidyl methacrylate with an amine-oxide-containing compound having a group reactive to epoxy group such as hydroxyethyl-N,N-dimethylamine oxide; and reaction product obtained by reacting an isocyanate-group containing monomer such as 2-isocyanateethyl methacrylate with an amine-oxide-containing compound having a group reactive to isocyanate group such as hydroxyethyl-N,N-dimethylamine oxide.

The monomers represented by the formula (b5-2) include amine-oxide-modified products of alkyl-, aryl- and alkylaryl-group-added products, which are exemplified by 2-vinylpyridine, 3-vinylpyridine, 4-vinylpyridine, 2-methyl-5-vinylpyridine, 3-methyl-5-vinylpyridine, 4-methyl-5-vinylpyridine, 6-methyl-5-vinylpyridine, 2-methyl-4-vinylpyridine, 3-methyl-4-vinylpyridine, 2-lauryl-5-vinylpyridine, 2-lauryl-4-vinylpyridine, 2-(*t*-butyl)-5-vinylpyridine, 2-(*t*-butyl)-4-vinylpyridine.

The monomers represented by the formula (b5-3) include amine-oxide-converted products of 1-vinylimidazole, 2-methyl-1-vinylimidazole, 4-methyl-1-vinylimidazole, 5-methyl-1-vinylimidazole, 2-lauryl-1-vinylimidazole and 4-(t-butyl)-1-vinylimidazole.

The monomers represented by the formula (b5-4) include amine-oxide-converted products of 4-vinylmorpholine, 2-methyl4-vinylmorpholine, 4-arylmorpholine, 1-vinylpiperidine, 4-methyl-4-vinylpiperidine, 2-lauryl-1-vinylpiperazine and 4-methylpiperazinoethyl methacrylate.

Among these, the monomers represented by the formula (b5-1) are most preferable as the monomer of the amine-oxide-group-containing polymer (b5), and in particular, (meth)acryloyloxyalkylene compound in which $R^{b2}$ and $R^{b3}$ are $C_{1-4}$ alkyl groups is most preferable.

The nitrogen-containing monomer capable of yielding the amine-oxide-group-containing monomer (i) or any structure derived therefrom by the above-described methods ① to ④ can be exemplified by the monomers represented by the formulae (b5-5) to (b5-8) below:

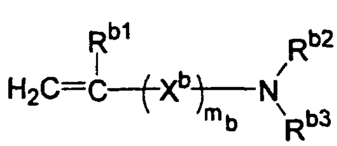

(b5-5)

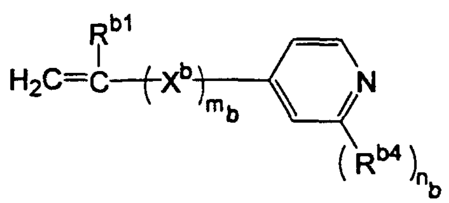

(b5-6)

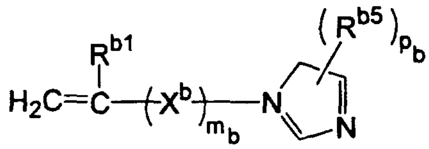

(b5-7)

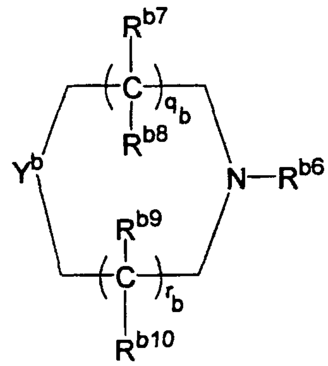

(b5-8)

where definitions for $R^{b1}$ to $R^{b10}$, $q_b$, $r_b$, $m_b$, $n_b$, $P_b$, $X^b$ and $Y^b$ are respectively same as those in the formulae (b5-1) to (b5-4).

A portion of the amine-oxide-group-containing monomer (b5) not exceeding 30% by weight thereof may be replaced by other hydrophilic monomer (iii).

The hydrophilic monomer (iii) can be exemplified by nonionic, anionic, cationic, or amphoteric monomers having both of an anionic and cationic ionicity in one molecule.

Of these, specific examples of the nonionic monomer include hydrophilic monomers such as those derived from (meth)acrylic acid or (meth)acrylamide reacted with a $C_{2-4}$ alkylene oxide, and (meth)acrylamide, which are exemplified by (meth)acrylonitrile, N-cyclohexylmaleimide, N-phenylmaleimide, N-vinylpyrrolidone, N-vinylformamide, N-vinylacetamide, N-(meth)acryloylmorpholine, hydroxyethyl (meth)acrylate, polyethylene glycol (meth)acrylate, methoxypoly(ethylene glycol/propylene glycol)mono(meth)acrylate, polyethylene glycol di(meth)acrylate and N-polyoxyalkylene (meth)acrylamide.

In this embodiment relating to a cosmetic polymer composition, ratio by weight ((a)/(b5)) of the block copolymer (a) and the amine-oxide-group-containing polymer (b5) is preferably falls in a range from 1/10 to 10/1, and more preferably from 1/5 to 10/1. The ratio of (a)/(b5) less than 1/10 may fail in keeping a desired hair style due to a poor styling effect, and may result in a shortage of elasticity of the hair under high humidity. On the other hand, the ratio exceeding 10/1 may result in a poor slipping nature when applied to the hair, and produces a feel of creaking.

This embodiment relating to a cosmetic polymer composition preferably contains the component (a) and component (b5) in a total amount of 0.1 to 10% by weight of the entire composition, and more preferably 0.5 to 8% by weight. The total amount of less than 0.1% by weight tends to result in only an insufficient styling power, and exceeding 10% by weight may increase the stiff touch and therefore worsen the feel of touch.

VII. Silicone Derivative

Another embodiment of the present invention relates to a composition containing, together with the block copolymer (a), a silicone derivative (b6). This embodiment is preferable as a hair cosmetic polymer composition. The silicone derivative is preferably contained in an amount of 0.01 to 50% by weight of the entire composition, and more preferably 0.1 to 20% by weight. The amount of addition of the silicone derivative less than 0.01% by weight may result in only an insufficient effect of addition, and exceeding 50% by weight may undesirably destabilizes the system.

The silicone derivative (b6) is preferably exemplified by poly(di-substituted siloxane) represented by the formula (b6-1):

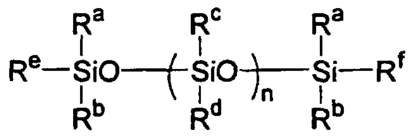

(b6-1)

where, $R^a$, $R^b$, $R^e$ and $R^f$ independently represent a methyl group or phenyl group; $R^c$ and $R^d$ independently represent a methyl group, phenyl group, aminoalkyl group or polyether group; and n is an integer from 3 to 20,000.

Among these, those preferably used include dimethylpolysiloxane derivatives represented by the formulae (b6-2) to (b6-7) below:

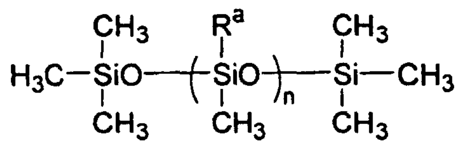

(b6-2)

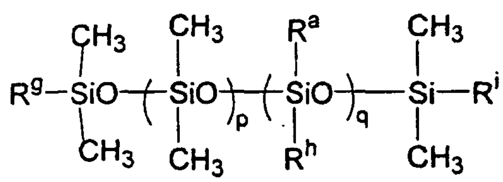

(b6-3)

where, p and q respectively represent an integer of one or larger (where p+q is 1 to 500); $R^a$ is defined same as that in the formula (b6-1) in the above; and $R^g$, $R^h$ and $R^i$ respectively represent any one functional group selected from the group consisting of methyl group, phenyl group, $-C_rH_{2r}O-(C_2H_4O)_s-(C_3H_6O)_t-A$, $-(CH_2)_u-B$, $-(CH_2)_2-CF_3$ and $-C_vH_{2v}-C_6H_5$, which may be identical or may differ from each other, where A represents a hydrogen atom or $C_{1-28}$ alkyl group, B represents an oxylane ring, r is an integer from 1 to 6, s and t respectively represent integers from 0 to 50, u is an integer from 1 to 3, and v is an integer from 0 to 4.

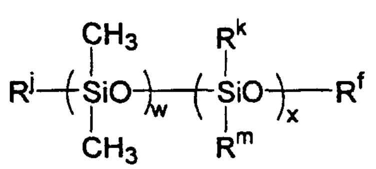

(b6-4)

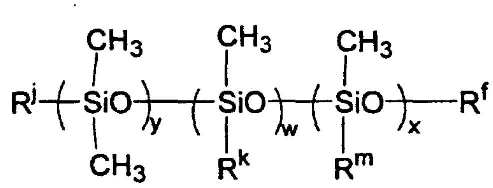

(b6-5)

where in two above formulae, $R^j$ represents a methyl group or hydroxyl group; $R^f$ is defined same as that in the formula (6b-1) in the above; $R^k$ represents any groups represented by the formulae (b6-6) or (b6-7) below; $R^m$ represents a hydroxyl group, hydroxyalkyl group, oxyalkylene group, or polyoxyalkylene group; and w, x and y respectively represent an integer depending on the molecular weight of the derivative.

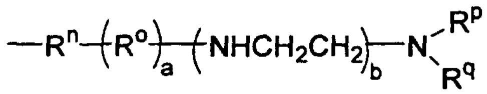

(b6-6)

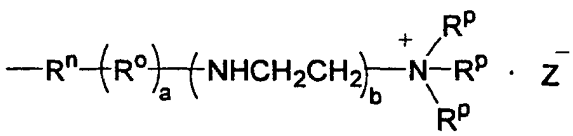

(b6-7)

where in two above formulae, $R^n$ represents a divalent hydrocarbon group; $R^o$ represents $-OCH_2CH_2-$, $-OCH(CH_3)CH_2-$, $-OCH_2CH(OH)CH_2-$ or $-OCH_2CH(CH_2OH))-$; $R^p$ and $R^q$ respectively represent a hydrogen atom or monovalent hydrocarbon group; a and b respectively represent an integer from 0 to 6; and $Z^-$ represents a halogen ion or organic anion.

Of these silicone derivatives, dimethylpolysiloxanes represented by the formulae (b6-2) and (b6-3) shown in the above, or polyether-modified silicone derivative is particularly preferable.

In this embodiment relating to a cosmetic polymer composition, the block copolymer (a) is used in an amount of addition of 0.01 to 20% by weight, and the silicone derivative (b) in an amount of 0.01 to 50% by weight.

The amount of use of the block copolymer (a) less than 0.01% by weight may fail in keeping a desired hair style due to a poor styling effect, and may result in a reduced elasticity of the hair under high humidity. On the other hand, the ratio exceeding 20% by weight may add a stiff touch and worsen the feel of touch. On the other hand, the amount of use of the silicone derivative (b) less than 0.01% by weight may result in a poor slipping nature when applied to the hair, and this makes combing difficult, and produces a feel of creaking and the amount of use exceeding 50% by weight may degrade the rinsing property after hair wash and may produce a tacky touch of hair.

This embodiment relating to a cosmetic polymer composition preferably has a number-average molecular weight of relatively as small as $5\times10^3$ to $5\times10^5$ for an expected use as a hair styling material, and relatively as large as $1\times10^4$ to $1\times10^6$ for an expected use as a hair conditioner.

For the purpose of use of the cosmetic polymer composition of this embodiment as a cosmetic material, the amount of addition of the block copolymer (a) adjusted to 0.1 to 10% by weight of the entire cosmetic material is successful in obtaining various cosmetic materials, in particular hair cosmetic material.

## VIII. Cosmetic Material

The cosmetic polymer composition of the present invention is applicable to cosmetic materials of various purposes. Among these, hair cosmetic material, skin cosmetic material and nail cosmetic material are preferable applications. The block copolymer can be provided as a polymer showing various characteristics required for various cosmetic materials, by determining composition of the individual blocks, for example, taking a hard/soft balance, and a hydrophilicity/hydrophobicity balance into consideration. For example, a cosmetic material containing the block copolymer composed of a hydrophilic segment, high-strength segment and high-elasticity segment can provide a hair cosmetic material having a desirable styling performance, no fear of becoming tacky under a moist condition nor becoming stiff under a dry condition, and a good hair washability which ensures easy-removing property with water. By similarly adjusting segment composition of the block copolymer and composition of each segment, the block copolymer can be provided also as a skin cosmetic material capable of uniformly spreading over the skin, being felt comfortable to the skin, and allowing medically active agent or the like to infuse through the skin. By similarly adjusting segment composition of the block copolymer and composition of each segment, the block copolymer can be provided also as a nail cosmetic material capable of uniformly spreading over the nail, readily forming a film, and ensuring an excellent film-retaining property.

The individual cosmetic materials will be detailed in the next.

### VIII-1 Hair cosmetic Material

The cosmetic polymer composition of the present invention is applicable to hair cosmetic materials of various purposes, such as shampoo, rinse, treatment, styling agent and permanent wave liquid and the like, and is also applicable in any form of liquid, cream, emulsion, gel, mousse or the like. For example, the block copolymer can be added to publicly-known hair cosmetic materials such as shampoo, rinse, treatment, styling agent and permanent wave liquid,

in place of any publicly-known polymers. It is also allowable to use the block copolymer together with the publicly-known polymer of conventional use. Although a preferable range of content of the block copolymer in the hair cosmetic material may differ depending on forms and purposes of the hair cosmetic material, and also on types and amount of other materials to be used in combination, the block copolymer is preferably contained in an amount of 0.01 to 10% by weight of the hair cosmetic material.

The next paragraphs will detail embodiments of the present invention applied to the hair cosmetic materials.

VIII-1-1 Styling Agent

The word "styling agent" in the context of this specification is used in a broad sense which covers all hair cosmetic materials used for hair styling, where any types of aerosol spray, pump-type hair spray, foam-type hair spray, hair mist, styling lotion, hair cream and hair oil are included. The styling agent is generally prepared by dissolving and/ or dispersing a polymer having a styling performance in a solvent such as water and/or alcohols such as ethanol and isopropanol. In the present invention, the above-described block copolymer can be used as the polymer having a styling performance in a singular manner, or in combination with publicly-known general styling polymers of cationic, anionic, nonionic or amphoteric type.

Publicly-known cationic polymers applicable to hair styling include ether formed by hydroxycellulose and glycidyl trimethylammonium chloride (Leoguard G, trade name, product of Lion Corporation)), Polymer JR-30M-125 and -400 (trade names, products of Union Carbide)), quaternized product of vinylpyrrolidone-dimethylaminoethyl methacrylate copolymer (Gafquat 734 and 755, trade names, products of GAF)), dimethyldiallylammonium chloride polymer (Merquat 100, trade name, product of Merck), and dimethyldiallylammonium chloride acrylamide copolymer (Merquat 550, trade name, product of Merck). Anionic polymers for the same purpose include copolymer of (meth)acrylate and alkyl methacrylate (Diahold, trade name, product of Mitsubishi Chemical Corporation), Plascize (trade name, product of Goo Chemical Co., Ltd.); and copolymer of monoalkyl maleate and methyl vinyl ether (trade name, Gantrez (product of ISP)). The nonionic polymers can be exemplified by polyvinylpyrrolidone polymer (PVP, trade name, product of ISP), and copolymer of vinylpyrrolidone and vinyl acetate (Luviskol, trade name, product of BASF AG)). The amphoteric polymers can be exemplified by methacrylic ester copolymer (Yukaformer AM-75W, trade name, product of Mitsubishi Chemical Corporation).

In an embodiment of hair cosmetic material (mousse) which can be injected in a foam state, the composition thereof is preferably adjusted so that the block copolymer accounts for 0.01 to 10% by weight, publicly-known styling polymer for 0 to 15% by weight, nonionic surfactant for 0.1 to 5% by weight, liquefied gas for 3 to 25% by weight, and water-soluble solvent mainly composed of water for 60% by weight to the residual portion. It is preferable herein that the hair cosmetic material is prepared so as to contain water in a content of 60% by weight or above. For an embodiment of gel, the composition thereof is preferably adjusted so that the block copolymer accounts for 0.01 to 10% by weight, publicly-known styling polymer for 0 to 15% by weight, gel base for 0.1 to 3% by weight, and water for 72% by weight to the residual portion. For an embodiment of hair spray, the composition thereof is preferably adjusted so that the block copolymer accounts for 0.01 to 10% by weight, publicly-known styling polymer for 0 to 15% by weight, solvent for 30 to 80% by weight, and injection agent for 10 to 70% by weight.

The nonionic surfactant applicable to hair mousse include sorbitan fatty acid ester, glycerin fatty acid ester, polyoxyethylenesorbitan fatty acid ester, polyethylene glycol fatty acid ester, polyoxyethylenealkyl ether, polyoxyethylenealkyl phenyl ether, polyoxyethylene castor oil, hardened polyoxyethylene castor oil and fatty acid alkanolamide. The injection aid applicable to spray or mousse include liquefied gases such as liquefied petroleum gas, dimethyl ether and halogenated hydrocarbon; and compressed gas such as air, carbon dioxide gas and nitrogen gas.

VIII-1-2 Conditioning Function Providing Agent

The word "conditioning function providing agent" in the context of this specification is used in a broad sense which covers all hair cosmetic materials used for hair conditioning, where any product forms of shampoo, rinse and permanent wave liquid are included. The hair cosmetic materials include those using, as a solvent, water and/or alcohols such as ethanol and isopropanol, which are exemplified by shampoo, rinse and permanent wave liquid; and those using, as a solvent, alcohols such as ethanol and isopropanol, or alcohols and/or hydrocarbons having a boiling point from 50°C to 300°C, which are exemplified by hair treatment. Similarly to the case of the above-described styling agent, the block copolymer may be used as a polymer having a conditioning performance in an singular manner, or in combination with generally-used conditioning polymers of cationic, anionic, nonionic or amphoteric type.

In an embodiment of shampoo, it is prepared by adding the block copolymer to any publicly-known anionic, amphoteric or nonionic surfactant base. The anionic surfactant base can be exemplified by N-(fatty acid) acyl-N-methyl-β-alanine salt such as N-coconyl-N-methyl-β-alanine sodium salt and N-myristoyl-N-methyl-β-alanine sodium salt; the amphoteric surfactant base is exemplified by cocoacidpropyl betaine, dimethyllauryl betaine, bis(2-hydroxyethyl)lauryl

betaine, cyclohexyllaurylamine oxide, dimethyllaurylamine oxide and bis(2-hydroxyethyl)laurylamine oxide; and the nonionic surfactant base is exemplified by stearic acid diethanol amide, palm oil fatty acid diethanol amide, sorbitan sesquioleate and polyoxyethylene stearyl ether.

In an embodiment of rinse, it is prepared by adding the block copolymer to any publicly-known cationic surfactant base. The cationic surfactant base is exemplified by stearyl trimethyl ammonium chloride, distearyl dimethyl ammonium chloride and stearyl dimethyl benzyl ammonium chloride.

In an embodiment of permanent wave liquid, it is prepared by adding the block copolymer to any publicly-known oxidizing agents such as bromate salts and perboric acid, and reducing agents such as thioglycolic acid and its salt, and cysteine.

In an embodiment of hair treatment, it is prepared by adding the block copolymer in combination with, or in place of any publicly-known cationic surfactant base, and/or cationic polymers such as cationic polypeptide, cationic cellulose and cationic polysiloxane. As the cationic surfactant base for hair treatment, those listed typically for the rinse are also available.

The hair cosmetic materials in either embodiment of styling agent and conditioning function providing agent can be added with, if necessary, other arbitrary component, besides the foregoing various components within a range not affective to the effects of the present invention. Examples of the arbitrary component include hydrocarbons such as liquid paraffin, vaseline, solid paraffin, squalane and olefin oligomer; straight-chain alcohols such as ethanol, lauryl alcohol, cetyl alcohol, stearyl alcohol, behenyl alcohol, myristyl alcohol, oleyl alcohol and cetostearyl alcoho; branched alcohols such as monostearylglycerin ether, 2-decyltetradecinol, lanolin alcohol, cholesterol, phytosterol, hexyl dodecanol, isostearyl alcohol and octyl dodecanol; higher fatty acids and their derivatives such as lauric acid, myristic acid, palmitic acid, stearic acid, behenic (behenyl) acid, oleic acid, 1,2-hydroxystearic acid, undecylenic acid, tall oil fatty acid, lanolin fatty acid, isostearic acid, linolic acid, linoleinic acid, γ-linolenic acid and eicosapentaenoic acid; water-soluble natural polymer including plant-origin polymers such as carrageenan, pectin, agar, quince seed (*Cydonia oblonga*), algae colloid (brown algae extract), starch (rice, corn, potato, wheat) and glycyrrhizinic acid; microbe-origin polymers such as xanthan gum, dextran, pullulan; and animal-origin polymers such as collagen and gelatin; cellulosic polymers such as methylcellulose, ethylcellulose, methylhydroxypropylcellulose, hydroxyethylcellulose, sodium cellulose sulfate, hydroxypropylcellulose, sodium carboxymethylcellulose (CMC), crystalline cellulose and cellulose powder; semi-synthetic, water-soluble polymers such as sodium arginate and propylene glycol alginate ester; vinyl-base polymers such as polyvinyl alcohol, polyvinyl methyl ether, polyvinyl pyrrolidone, carboxyvinyl polymer (Carbopol); polyoxyethylene-base polymers such as polyethylene glycols 20,000, 4,000,000 and 600,000; synthetic water-soluble polymers such as polyethyleneimine; inorganic water-soluble polymers such as bentonite, AlMg silicate (Veegum), labonite, hectorite and silisic anhydride; silicones such as volatile silicone oil, silicone polymer, silicone rubber and alkyl-modified silicone; N-(fatty acid)acyl-L-glutamate salt such as monosodium N-lauryl-L-glutamate, N-(palm oil fatty acid)-L-glutamate monotriethanolamine, monosodium N-myristic acid acyl-L-glutamate, monosodium N-(mixed fatty acid)acyl-L-glutamate; N-(fatty acid)-N-methyltaurine salt such as lauric acid methyltaurine sodium salt and palm oil fatty acid methyltaurine sodium salt; salts of N- (fatty acid) sarcosine condensate such as sodium lauroylsarcosinate and sodium cocoyl sarcosinate; surfactants such as sodium acylsarcosinate, sodium acylglutamate, sodium acyl-β-alanine, acyl taurate, lauryl sulfate, lauryl dimethylaminoacetate betaine, alkyl trimethyl ammonium chloride and polyoxyethylene-hardened castor oil; sequestering agent such as 1-hydroxyethane-1,1-diphosphonic acid, 1-hydroxyethane-1,1-diphosphonic acid tetrasodium salt, EDTA disodium salt, EDTA trisodium salt, EDTA tetrasodium salt, sodium citrate, sodium polyphosphate, sodium metaphosphate and gluconic acid; various UV absorbing agent such as 3-(4'-methylbenzylidene)-d,1-camphor, 3-benzylidene-d,1-camphor, urocanic acid, urocanic acid ethyl ester, 2-phenyl-5-methylbenzoxazole, 2,2'-hydroxy-5-methylphenylbenzotriazole, 2-(2'-hydroxy-5'-*t*-octylphenyl)benzotriazole, 2-(2'-hydroxy-5'-methylphenylbenzotriazole, dibenzalazine, dianisoylmethane, 4-methoxy-4'-*t*-butyldibenzoylmethane, 5-(3,3-dimethyl-2-norbornylidene)-3-pentane-2-one, and those of benzoic acid-base, anthranilic acid-base, salicylic acid-base, cinnamic acid-base and benzophenone-base; emulsifying agent such as glyceryl monostearate, seryl monostearate, sorbitan monopalmitate, polyoxyethylene cetyl ether and polyoxyethylene sorbitan monolaurate; moisturizers such as (poly)ethylene glycol, (poly)propylene glycol, glycerin, 1,3-butylene glycol, maltitol, sorbitol, chondroitin sulfate, hyaluronic acid, atelocollagen, cholesteryl-1,2-hydroxystearate, sodium lactate, bile acid salt, dl-pyrrolidone carboxylate salt and short-chain soluble collagen; antibacterial agent such as hinokitiol, hexachlorophene, benzalkonium chloride, trichlorocarbanilide and pitionol; vasodilators such as carpronium chloride; refreshing agent such as menthols; stimulator such as benzyl nicotinate; vitamins such as vitamin A, B, C, D and E; disinfectant/antiseptic agent such as chlorhexidine gluconate, isopropyl methyl phenol and *p*-oxybenzoate ester; chelating agent such as protein hydrolysate, amino acid, plant extract and EDTA-Na; pH adjuster such as succinic acid, sodium succinate and triethanolamine; foam increasing agent; foaming agent; foam stabilizer; injection agents for use in aerosol product, such as liquefied petroleum gas and dimethyl ether; sequestering agent; mildewproof agent; emulsifier; conditioner; thickener; antioxidant; solubilizing agent; rosin; hydrotrope; hair tonic; crude drugs; dye; and Flavoring ingredient.

VIII-2. Skin and Nail Cosmetic Materials

Forms of the skin and nail cosmetic materials are not specifically limited, where the skin cosmetic materials include basic cosmetic materials such as cream and milky lotion, and make-up materials such as foundation, face powder, cheek color, eyeshadow and lip color. Nail cosmetic materials include nail color, nail care cream, nail enamel, base coat for nail enamel, and top coat for nail enamel.

In embodiments of the skin care and nail care cosmetic materials, the block copolymer is preferably contained in the cosmetic material in an amount of 1 to 30% by weight. The skin care and nail care cosmetic materials can be manufactured by dissolving, emulsifying or dispersing a block-copolymer-containing component to be blended into any of water, alcoholic solvents such as ethanol, ester-base solvent such as ethyl acetate, ketone-base solvent such as methyl ethyl ketone, and hydrocarbons such as liquid paraffin and vaseline.

Examples

The following paragraphs will specifically describe the present invention referring to exemplary manufacture and embodiments, where it is to be understood that the present invention is by no means limited to these exemplary manufacture and embodiments, without departing from the spirit of the invention. It is to be noted that descriptions of "part" and "%" in the exemplary manufacturing and embodiments are expressed on the weight basis unless otherwise specified.

In the following examples, molecular weight and molecular weight distribution were measured by GPC through a polystyrene gel column using tetrahydrofuran as a mobile phase, and determined on polystyrene basis.

The glass transition temperature was measured by DSC (differential scanning calorimetry) at a temperature elevation speed of 20°C/min conforming to JIS K7121.

[Exemplary Manufacture 1] (Exemplary Manufacture of 2-Ethylhexyl Acrylate/t-Butyl Acrylate-Base Block Copolymer P-1)

In a nitrogen-replaced reaction vessel equipped with a thermocouple and a stirring propeller, 430 mg of copper (I) bromide was placed and then heated to 80°C. The content of the reaction vessel, under the nitrogen atmosphere and stirring at 250 rpm, was further added with a mixed solution containing 1.37 g dimethyl-2,6-dibromoheptane dioate, 184 g of 2-ethylhexyl acrylate, 1.04 g of pentamethyldiethylene triamine and 90 g of dimethylformamide. After stirred for 3 hours, the reaction vessel was quickly cooled in an ice bath to thereby terminate the reaction. The reaction solution was poured into a MeOH/water (5/1) mixed solvent, washed, the supernatant was removed by decantation, the deposited polymer was dissolved in toluene, added with water to thereby separate the solution into a polymer phase and a catalyst phase so as to remove copper bromide, the solvent in the polymer phase was removed by reduced-pressure distillation, and the residue was dried. The resultant polymer (occasionally referred to as "poly 2-ethylhexylacrylate macro-initiator", hereinafter) was found to have a weight-average molecular weight (Mw) of 10,800, number-average molecular weight (Mn) of 10, 000, and molecular weight distribution (Mw/Mn) of 1.07.

In a nitrogen-replaced reaction vessel equipped with a thermocouple and a stirring propeller, 715 mg of copper (I) bromide was placed and then heated to 80°C. The content of the reaction vessel, under the nitrogen atmosphere and stirring at 250 rpm, was further added with a mixed solution containing 50 g of poly 2-ethylhexylacrylate macro-initiator, 320 g of *t*-butyl acrylate, 1.735 g of pentamethyl diethylene triamine, and 138 g of dimethylformamide. After stirred for 5 hours, the reaction vessel was quickly cooled in an ice bath to thereby terminate the reaction. The reaction solution was poured into a MeOH/water (5/1) mixed solvent, washed, the supernatant was removed by decantation, the deposited polymer was dissolved in toluene, added with water to thereby separate the solution into a polymer phase and a catalyst phase so as to remove copper bromide, the solvent in the polymer phase was removed by reduced-pressure distillation, and the residue was dried. The resultant polymer was found to have a weight-average molecular weight (Mw) of 49,800, number-average molecular weight (Mn) of 37, 500, and molecular weight distribution (Mw/Mn) of 1.33. Weight fractions of 2-ethylhexyl acrylate and *t*-butyl acrylate in the block copolymer calculated from Mn were found to be 27% by weight and 73% by weight, respectively. The weight fractions of 2-ethylhexyl acrylate and *t*-butyl acrylate in the block copolymer were also confirmed by $^1$H-NMR.

The obtained block copolymer was a triblock copolymer.

Seventy grams of thus obtained triblock copolymer of 2-ethylhexyl acrylate and *t*-butyl acrylate was dissolved into 650 ml of toluene. The mixture was then added with 3.8 g of *p*-toluenesulfonic acid, and refluxed under heating in an oil bath at 100°C for 2 hours. The mixture was then cooled, the solvent was condensed under reduced pressure, the residue was dissolved in tetrahydrofuran and re-precipitated in water, and the precipitate was dried under reduced pressure to thereby obtain block copolymer P-1.

Ratio of hydrolysis of thus obtained block copolymer P-1 was confirmed by acid value titration using a 0.1

mol/L aqueous potassium hydroxide solution, and was found to be 100%. Glass transition temperature (Tg) was found to be -50°C for the 2-ethylhexyl acrylate block, and 107°C for the acrylic acid block. Compositional ratio of the obtained block copolymer was confirmed by $^{13}$C-NMR measurement, by which weight fractions of 2-ethylhexyl acrylate and acrylic acid were found to be 45% by weight and 55% by weight, respectively.

[Exemplary Manufacture 2] (Exemplary Manufacture of 2-Ethylhexyl Acrylate/t-Butyl Acrylate-Base Block Copolymer P-2)

Similarly to the manufacturing process described in the above, the reaction was proceeded for 1 hour using 1.29 g of copper (I) bromide, 4.11 g of dimethyl-2,6-dibromoheptane dioate, 553.2 g of 2-ethylhexyl acrylate, 3.12 g of pentamethyl diethylene triamine, and 280 g of dimethylformamide. The obtained polymer was found to have a weight-average molecular weight (Mw) of 13,800, number-average molecular weight (Mn) of 12,800, and a molecular weight distribution (Mw/Mn) of 1.08.

In an exemplary manufacture similarly to as described in the above, the polymerization was similarly proceeded using 2.15 g of copper (I) bromide, 200 g of poly 2-ethylhexylacrylate macro-initiator, 1,000 g of *t*-butyl acrylate, 5.2 g of pentamethyl diethylene triamine, and 370 g of dimethylformamide, to thereby obtain the block copolymer.

The resultant polymer was found to have a weight-average molecular weight (Mw) of 68,600, number-average molecular weight (Mn) of 51,500, and molecular weight distribution (Mw/Mn) of 1.33. Weight fractions of 2-ethylhexyl acrylate and *t*-butyl acrylate in the block copolymer calculated from Mn were found to be 25% by weight and 75% by weight, respectively.

The copolymer was then hydrolyzed under the conditions similar to those described in the above, to thereby obtain block copolymer P-2. Ratio of hydrolysis was similarly determined, by which the block copolymer P-2 was found to have a ratio of hydrolysis of 100%. The glass transition temperature (Tg) was found to be -50°C for the 2-ethylhexyl acrylate block, and 107°C for the acrylic acid block. Compositional ratio of the obtained block copolymer was confirmed by $^{13}$C-NMR measurement, by which weight fractions of 2-ethylhexyl acrylate and acrylic acid were found to be 40% by weight and 60% by weight, respectively.

[Exemplary Manufacture 3] (Exemplary Manufacture of 2-Ethylhexyl Acrylate/t-Butyl Acrylate-Base Block Copolymer P-3)

In a nitrogen-replaced reaction vessel equipped with a thermocouple and a stirring propeller, 550 mg of copper (I) bromide was placed and then heated to 100°C. The content of the reaction vessel, under the nitrogen atmosphere and stirring at 250 rpm, was further added with a mixed solution containing 2.65 g dimethyl-2,6-dibromoheptane dioate, 354 g of 2-ethylhexyl acrylate, 885 mg of 2,5,8,11-tetramethyl-2,5,8,11-tetraazadodecane and 200 g of toluene. After stirring for 3 hours, the reaction vessel was quickly cooled in an ice bath to thereby terminate the reaction. The reaction solution was poured into a MeOH/water (5/1) mixed solvent, washed, the supernatant was removed by decantation, the deposited polymer was dissolved in toluene, added with water to thereby separate the solution into a polymer phase and a catalyst phase so as to remove copper bromide, the solvent in the polymer phase was removed by reduced-pressure distillation, and the residue was dried. The resultant polymer was found to have a weight-average molecular weight (Mw) of 39,800, number-average molecular weight (Mn) of 20,000, and molecular weight distribution (Mw/Mn) of 1.99.

In a nitrogen-replaced reaction vessel equipped with a thermocouple and a stirring propeller, 826 mg of copper (I) bromide was placed and then heated to 100°C. The content of the reaction vessel, under the nitrogen atmosphere and stirring at 250 rpm, was further added with a mixed solution containing 50 g of poly 2-ethylhexylacrylate macro-initiator, 265 g of *t*-butyl acrylate, 1.32 g of 2,5,8,11-tetramethyl-2,5,8,11-tetraazadodecane and 150 g of toluene. After stirred for 5 hours, the reaction vessel was quickly cooled in an ice bath to thereby terminate the reaction. The reaction solution was poured into a MeOH/water (5/1) mixed solvent, washed, the supernatant was removed by decantation, the deposited polymer was dissolved in toluene, added with water to thereby separate the solution into a polymer phase and a catalyst phase so as to remove copper bromide, the solvent in the polymer phase was removed by reduced-pressure distillation, and the residue was dried, o thereby obtain the block copolymer.

The obtained block copolymer was found to have a weight-average molecular weight (Mw) of 83,700, number-average molecular weight (Mn) of 39,500, and molecular weight distribution (Mw/Mn) of 2.12. Weight fractions of 2-ethylhexyl acrylate and *t*-butyl acrylate in the block copolymer calculated from Mn were found to be 50% by weight and 50% by weight, respectively. Weight fraction of the obtained block copolymer was confirmed also by $^{1}$H-NMR measurement. The obtained block copolymer was a triblock copolymer.

The copolymer was then hydrolyzed under the conditions similar to those described in the above, to thereby obtain block copolymer P-3. Ratio of hydrolysis of the block copolymer P-3 was found to be 100%. The glass transition temperature (Tg) was found to be -50°C for the 2-ethylhexyl acrylate block, and 107°C for the acrylic acid block.

Compositional ratio of the obtained block copolymer was confirmed by $^{13}$C-NMR measurement, by which weight fractions of 2-ethylhexyl acrylate and acrylic acid were found to be 45% by weight and 55% by weight, respectively.

[Exemplary Manufacture 4] (Exemplary Manufacture of 2-Ethylhexyl Acrylate/t-Butyl Acrylate-Base Block Copolymer P-4)

A reaction vessel was attached with a thermocouple and a stirring propeller, the inner atmosphere of which was replaced with nitrogen, 173 mg of copper (I) bromide was placed therein, and the content was heated to 80°C. While keeping the nitrogen atmosphere inside the reaction vessel and stirring at 250 rpm, a mixed solution containing 697 mg dimethyl-2,6-dibromoheptane dioate, 184 g of 2-ethylhexyl acrylate, 419 mg of pentamethyl diethylene triamine and 88 g of dimethylformamide was added to the content of the reaction vessel. After stirred for 2 hours, the reaction vessel was quickly cooled in an ice bath to thereby terminate the reaction. The reaction solution was added with a mixed solution of tetrahydrofuran and water so as to be separated into a polymer phase and a catalyst phase, copper bromide was removed, the polymer phase was dropped into a large amount of methanol to thereby allow the polymer to re-precipitate, and the solvent was removed by filtration. The obtained poly 2-ethylhexylacrylate macro-initiator was found to have a weight-average molecular weight (Mw) of 22,600, number-average molecular weight (Mn) of 12, 000, and molecular weight distribution (Mw/Mn) of 1.87.

A reaction vessel was attached with a thermocouple and a stirring propeller, the inner atmosphere of which was replaced with nitrogen, 717 mg of copper (I) bromide and 58.3 mg of copper (II) bromide were placed therein, and the content was heated to 80°C. While keeping the nitrogen atmosphere inside the reaction vessel and stirring at 250 rpm, a mixed solution containing 30 g of the poly 2-ethylhexylacrylate macro-initiator obtained in the above, 151 g of *t*-butyl acrylate, 1 g of pentamethyl diethylene triamine and 66 g of dimethylformamide was added to the content of the reaction vessel. After stirred for 3 hours, the reaction vessel was quickly cooled in an ice bath to thereby terminate the reaction. The system was added with a mixed solution of tetrahydrofuran and water so as to be separated into a polymer phase and a catalyst phase, the polymer phase was allowed to pass through an aluminum silicatge column (Kyoward 700SN, product of Kyowa Chemical Industry Co., Ltd.) to thereby completely remove copper bromides, the eluate was dropped into a large amount of methanol to thereby allow the polymer to re-precipitate, and the solvent was removed by filtration. The obtained copolymer was found to have a weight-average molecular weight (Mw) of 45,000, number-average molecular weight (Mn) of 25,400, and molecular weight distribution (Mw/Mn) of 1.77. Weight fractions of 2-ethylhexyl acrylate and *t*-butyl acrylate in the block copolymer calculated from Mn were found to be 47% by weight and 53% by weight, respectively. Weight fraction of the obtained block copolymer was confirmed also by $^{1}$H-NMR measurement. The obtained block copolymer was a triblock copolymer.

Eighteen grams of thus obtained triblock copolymer of 2-ethylhexyl acrylate and *t*-butyl acrylate was dissolved into 500 ml of 1,4-dioxane. The mixture was then added with 36 ml of a 6-mol/L hydrochloric acid, and refluxed under heating in an oil bath at 120°C for 20 hours. The mixture was then cooled, the solvent was condensed under reduced pressure, the residue was allowed to re-precipitate in a large amount of hexane, and the solvent was removed by filtration. The obtained polymer was washed with a large amount of water, and dried under reduced pressure to thereby obtain block copolymer P-4.

Ratio of hydrolysis of thus obtained block copolymer P-4 was confirmed by acid value titration using a 0.1-mol/L aqueous potassium hydroxide solution, and was found to be 50%. Glass transition temperatures (Tg) were found to be -50°C ascribabel to 2-ethylhexyl acrylate block, 43°C ascribable to *t*-butyl acrylate block, and 107°C ascribable to acrylic acid block. These glass transition temperatures were found to nearly coincide with those of the individual homopolymers.

[Exemplary Manufacture 5] (Exemplary Manufacture of 2-Ethylhexyl Acrylate/t-Butyl Acrylate-Base Block Copolymer P-5)

A reaction vessel was attached with a thermocouple and a stirring propeller, the inner atmosphere of which was replaced with nitrogen, 165mg of copper (I) bromide was placed therein, and the content was heated to 80°C. While keeping the nitrogen atmosphere inside the reaction vessel and stirring at 250 rpm, a mixed solution containing 692 mg dimethyl-2,6-dibromoheptane dioate, 184 g of 2-ethylhexyl acrylate, 398 mg of pentamethyl diethylene triamine and 88 g of dimethylformamide was added to the content of the reaction vessel. After stirred for 3 hours, the reaction vessel was quickly cooled in an ice bath to thereby terminate the reaction. The reaction solution was added with a mixed solution of tetrahydrofuran and water so as to be separated into a polymer phase and a catalyst phase, copper bromide was removed, the polymer phase was dropped into a large amount of methanol to thereby allow the polymer to re-precipitate, and the solvent was removed by filtration. The obtained poly 2-ethylhexylacrylate macro-initiator was found to have a weight-average molecular weight (Mw) of 33,000, number-average molecular weight (Mn) of 24,000, and molecular weight distribution (Mw/Mn) of 1.38.

Another reaction vessel was attached with a thermocouple and a stirring propeller, the inner atmosphere of which was replaced with nitrogen, 28.6 mg of copper (I) bromide and 9. 33 mg of copper (II) bromide were placed therein, and the content was heated to 80°C. While keeping the nitrogen atmosphere inside the reaction vessel and stirring at 250 rpm, a mixed solution containing 48 g of the poly 2-ethylhexylacrylate macro-initiator obtained in the above, 128 g of t-butyl acrylate, 79.7 mg of pentamethyl diethylene triamine and 53 g of dimethylformamide was added to the content of the reaction vessel. After stirred for 2 hours, the reaction vessel was quickly cooled in an ice bath to thereby terminate the reaction. The system was added with a mixed solution of tetrahydrofuran and water so as to be separated into a polymer phase and a catalyst phase, the polymer phase was allowed to pass through an aluminum silicatge column (Kyoward 700SN, product of Kyowa Chemical Industry Co., Ltd.) to thereby completely remove copper bromides, the eluate was dropped into a large amount of methanol to thereby allow the polymer to re-precipitate, and the solvent was removed by filtration.

The obtained copolymer was found to have a weight-average molecular weight (Mw) of 56,000, number-average molecular weight (Mn) of 39,800, and molecular weight distribution (Mw/Mn) of 1.41.

Weight fractions of 2-ethylhexyl acrylate and t-butyl acrylate in the block copolymer calculated from Mn were found to be 60% by weight and 40% by weight, respectively. Weight fraction of the obtained block copolymer was confirmed also by $^1$H-NMR measurement. The obtained block copolymer was a triblock copolymer having a configuration of poly(*t*-BA)/poly(2EHA)/poly(*t*-BA).

Twenty-one grams of thus obtained triblock copolymer of 2-ethylhexyl acrylate and t-butyl acrylate was dissolved into 480 ml of 1,4-dioxane. The mixture was then added with 33 ml of a 6-mol/L hydrochloric acid, and refluxed under heating in an oil bath at 120°C for 6 hours. The mixture was then cooled, the solvent was condensed under reduced pressure, the residue was allowed to re-precipitate in a large amount of hexane, and the solvent was removed by filtration. The obtained polymer was washed with a large amount of water, and dried under reduced pressure to thereby obtain block copolymer P-5.

Ratio of hydrolysis of thus obtained block copolymer P-5 was confirmed by neutralization titration using a 0.1-mol/L aqueous potassium hydroxide solution, and was found to be 61%. Glass transition temperatures (Tg) measured as described below were found to be -50°C ascribabel to 2-ethylhexyl acrylate block, 43°C ascribable to *t*-butyl acrylate block, and 107°C ascribable to acrylic acid block. These glass transition temperatures were found to nearly coincide with those of the individual homopolymers.

[Exemplary Manufacture 6] (Exemplary Manufacture of 2-Ethylhexyl Acrylate/t-Butyl Acrylate-Base Block Copolymer P-6)

Senventeen grams of the triblock polymer composed of 2-ethylhexyl acrylate and *t*-butyl acrylate obtained in Exemplary Manufacture 4 was added to 450 mL of 1,4-dioxane. The solution was added with 6 mL of a 6 mol/L hydrochloric acid, and refluxed under heating in an oil bath at 120°C for 20 hours. The mixture was then cooled, the solvent was condensed under reduced pressure, the residue was allowed to re-precipitate in a large amount of hexane, and the solvent was removed by filtration. The obtained polymer was washed with a large amount of water, and dried under reduced pressure to thereby obtain block copolymer P-6.

Ratio of hydrolysis of thus obtained block copolymer P-6 was confirmed by acid value titration using a 0.1-mol/L aqueous potassium hydroxide solution, and was found to be 26%. Glass transition temperatures (Tg) measured as described below were found to be -50°C ascribabel to 2-ethylhexyl acrylate block, 43°C ascribable to *t*-butyl acrylate block, and 107°C ascribable to acrylic acid block. These glass transition temperatures were found to nearly coincide with those of the individual homopolymers.

[Exemplary Manufacture 7] (Exemplary Manufacture of Comparative Random Copolymer (Pc-1))

A five-necked flask is attached with a reflux condenser, a dropping funnel, a thermometer, a nitrogen-replacement glass tube and a stirrer, 60 parts by weight of 2-ethylhexyl acrylate, 40 parts by weight of acrylic acid and 100 parts by weight of ethanol were placed therein, 1.0 weight part of $\alpha,\alpha'$-azobis(isovaleronitrile) was added, and the mixture was refluxed at 80°C under nitrogen-replaced atmosphere for 10 hours so as to proceed polymerization. The obtained copolymer was neutralized by 25% using a 20% aqueous KOH solution. Glass transition point of the obtained copolymer was found to be -6 °C. The random copolymer was named as Pc-1. Pc-1 was found to have a weight-average molecular weight of 40,000, and molecular weight distribution (Mw/Mn) of 3.51.

Compositions, expressed in ratio by weight, of the individual copolymers obtained in the above were EHA/AA=45/55 for block copolymer P-1, EHA/AA=40/60 for block copolymer P-2, EHA/AA=45/55 for block copolymer P-3, EHA/TBA/AA=60/16/24 for block copolymer P-5, EHA/TBA/AA=47.2/26.3/26.5 for block copolymer P-4, EHA/TBA/AA=47.2/39.3/13.5 for block copolymer P-6, and EHA/AA=60/40 for random copolymer Pc-1, where EHA is an abbreviation for 2-ethylhexyl acrylate, TBA is for *t*-butyl acrylate, and AA for acrylic acid.

(Comparative Random Copolymer Pc-2)

A commercially-available, anionic random copolymer (maleic monoalkyl ester/methyl vinyl ether copolymer, Gantrez A-425 (trade name, product of ISP)) was additionally used as Pc-2.

[Exemplary Manufacture 8] (Exemplary Manufacture of Comparative Random Copolymer having Amine Oxide Group (Pc-3))

In a reaction vessel equipped with a reflux condenser, a dropping funnel, a thermometer, a nitrogen-replacement glass tube and a stirrer, 50 parts of N, N-dimethylaminoethyl methacrylate, 30 parts of methyl methacrylate, 20 parts of isobutyl methacrylate, and 150 parts of absolute ethanol were placed therein, 0.6 parts of 2,2'-azobis(isobutylonitrile) was added, the mixture was allowed to react at 80°C under nitrogen-replaced atmosphere for 8 hours, and then cooled to 60°C.

To the polymerization solution, a 31% aqueous solution of hydrogen peroxide in an equimolar amount with N,N-dimethylaminoethyl methacrylate was dropped over one hour, the mixture was further stirred for 20 hours so as to proceed oxide-forming reaction of dimetylamino group, and was added with 150 absolute ethanol to thereby adjust the polymer content to 30%. Completion of the oxide-forming reaction was confirmed by measurement of amine value of the reaction liquid. The obtained polymer was named as Pc-3. Pc-3 was found to have a weight-average molecular weight of 110,000. Production of amine oxide group was confirmed by observing absorption ascribable to N-O group in an IR spectrum.

[Exemplary Manufacture 9] (Exemplary Manufacture of Copolymer having Amine Oxide Group (Pc-4))

Similarly to Exemplary Manufacture 8 described in the above, 70 g of N,N-dimethylaminoethyl methacrylate, 30 g of stearyl methacrylate, and 150 g of absolute ethanol were placed in a reaction vessel, the mixture was further added with 0.6 g of 2,2'-azobis (isobutylonitride), the mixture was allowed to react at 80°C under nitrogen-replaced atmosphere for 8 hours, and then cooled to 60°C.

Thereafter, an amine-oxide-group containing polymer (30% concentration) was prepared similarly to as described in Exemplary Manufacture 8 in the above. The obtained polymer was named as Pc-4. Pc-4 was found to have a weight-average molecular weight of 110,000. Production of amine oxide group was confirmed by observing absorption ascribable to N-O group in an IR spectrum.

1. Example 1: Film Properties of Film and Various Charactertics as Hair cosmetic Material

1-1 Film Properties

Film properties (Young's modulus and elongation percentage) of the block copolymers P-1 to 4, and comparative polymers Pc-1 and 2 were measured.

First, 3% solutions of the individual polymers in EtOH/$H_2O$ (1/2) were prepared (adjusted to pH=7 with a 20% aqueous NaOH solution, except for P-3 and P-4 using a 20% aqueous KOH solution), and the individual polymer solutions were allowed to dry in a Teflon-made Petri dish under a condition of 23° C, 60% RH. The polymer solution after being filmed was further allowed to stand in the Teflon-made Petri dish until weight of the film would not vary anymore (approximately 10 days). Amount of the polymer solution was adjusted so as to give a thickness of the resultant film of 100±15 μm.

Thus obtained individual films were slit to produce strips of 5 mm wide and 40 mm long, and the strips were used as samples for tensile test. The tensile test was carried out under a condition of 23°C, 60% RH, which is same as the film drying condition, and under a tensile speed of 20 mm/min, a distance between clamp flanges of 20 mm, to thereby measure Young's modulus and fracture-point elongation percentage. Tensile test of the film (Yonng's modulus and elongation percentage) was carried out conforming to JIS K7161. Results of the measurement were shown in Table 1.

1-2 Evaluation as Hair cosmetic Material

Block copolymers P-1 to 4 and comparative polymers Pc-1 and 2 were neutralized using a 20% aqueous potassium hydroxide solution, and respectively added with a mixed solution of water and ethanol so as to adjust the concentration thereof to 3% by weight, to thereby prepare pump-type hair spray compositions. Each of thus obtained hair spray composition was applied to the hair, and evaluated for its styling ability, style-keeping performance, tackiness, flaking property, feeling of touch and washability according to the evaluation criteria shown below. Results were shown

in Table 1.

(1) Style-Keeping Ability (Styling Performance)

A hair yarn bundle of 23 cm in length and 2.0 g in weight was dipped in a 3% polymer solution, taken out, lightly sqeezed, wound around a 1-cm diameter rod, and allowed to dry. The hair yarn bundle was removed from the rod, and a curled sample was obtained ($L_0$ in length). The hair bundle was vertically hung for 3 hours in a thermo-hygrostat chamber preliminarily conditioned at 20°C, 90% RH over 3 hours, and then measured for its length ($L_2$), curl-retention ratio (%) was calculated from the equation below, and evaluated according to the following criteria.

$$\text{Curl-retention ratio (\%)}= [(23-L_2)/(23-L_0)\}\times 100$$

◎ : Curl-retention ratio of 85% to 100%
○: Curl-retention ratio of 60% or more and less than 85%
Δ : Curl-retention ratio of 30% or more and less than 60%
× : Curl-retention ratio of 0% or more and less than 30%

(2) Washability

The hair yarn bundle was processed similarly to as described in the style-keeping performance test described in the above, the resultant curled hair yarn bundle was allowed to stand under a thermo-hygrostatic condition of 23°C, 60% RH, dipped in a 10% aqueous solution of an anionic surfactant (sodium polyoxyethylene laurylsulfate) at 40°C for one hour, rinsed with warm water at 40°C, dried, and whether the polymer remains on the hair yarn bundle or not was checked by visual observation and finger touch, and evaluated according to the following criteria.

○: No residual polymer confirmed visually and tactually.
Δ : A slight amount of residual polymer visually confirmed.
× : Residual polymer visually confirmed.

(3) Evaluation of Tacky Touch of Hair

A hair yarn bundle of 15 cm in length and 10 g in weight was coated with approximately 2 g of each sample, and allowed to stand at room temperature. Thirty minutes after, the tacky touch was evaluated through sensory test by a ten-membered panel, according to the following criteria.

◎ : None of 10 panelists recognized tacky touch.
○: One ore two panelists recognized tacky touch.
Δ : Three to five panelists recognized tacky touch.
× : Seven or more panelists recognized tacky touch.

(4) Evaluation of Finger Touch of Hair

A hair yarn bundle of 15 cm in length and 10 g in weight was coated with approximately 2 g of each sample, and allowed to stand at room temperature. Thirty minutes after, the feel to the touch was evaluated through sensory test by a ten-membered panel, according to the following criteria.

◎ : All of ten panelists recognized that it was good to the touch.
○: Eight or more of ten panelists recognized that it was good to the touch.
Δ : Four to seven of ten panelists recognized that it was good to the touch.
× : Only one to three of ten panelists recognized that it was good to the touch.

(5) Styling Ability Test (Bending Strength of Hair Yarn Bundle)

A hair yarn bundle of 15 cm in length was coated with 0.7 g of each sample, immediately shaped in 2-cm width, dried, and allowed to stand for one hour in a thermo-hygrostat chamber conditioned at 23°C, 60% RH. The sample was then placed on the supports 65 mm distant from each other and bent at the center thereof under a constant speed, and maximum load was measured and evaluated according to the following criteria.

○: The maximum load was 200 g or above, and the coated hairs were soft to the touch and there was no incompatibility after measurement.
Δ : The maximum load was 100 g or more, and the coated hairs were soft to the touch, but there was a certain level of incompatibility after measurement.
× : The maximum load was less than 100 g, and the coated hairs were not soft to the touch, and there was incompatibility after measurement.

(6) Style-Keeping Ability Test (Hair Yarn Bundle Bending Test)

After the style-keeping performance test, maximum load after the hair yarn bundle was broken was evaluated according to the following criteria.

○: Maximum load was 15 g or more.
Δ : Maximum load was 10 g or more and less than 15 g.
× : Maximum load was less than 10 g.

(7) Flaking

A constant volume of each sample was sprayed to the hair yarn bundle, completely dried, combed, and the amount of dropped polymer flakes (state of flaking) remaining on the hair yarns was observed under a stereo-microscope (×20). Evaluation criteria are as shown below.

○: Peeled polymer not observed, no flaking.
Δ : A slight amount of peeled polymer observed, slight flaking
× : A lot of peeled polymer observed, considerable flaking.

(8) Hair Gloss

A hair yarn bundle of 15 cm in length was coated with 0. 7 g of each sample, immediately shaped in 2-cm width, dried, and allowed to stand for one hour in a thermo-hygrostat chamber conditioned at 23°C, 60% RH. The obtained sample was checked by a sensory test participated by a ten-membered panel, and gloss of the hair yarn bundle was evaluated according to the following criteria.

◎ : All of ten panelists recognized that hair-bundle gloss was good.
○: Eight or more of ten panelists recognized that hair-bundle gloss was good.
Δ : Four to seven of ten panelists recognized that hair-bundle gloss was good.
× : Only one to three of ten panelists recognized that hair-bundle gloss was good.

(9) Combing Smoothness

The hair yarn bundle was processed similarly to as described in the above, resistance felt during combing of the hair yarn bundle was checked by a sensory test participated by a ten-membered panel, and evaluated according to the following criteria.

◎ : All of ten panelists recognized that it was easy to comb.
○: Eight or more of ten panelists recognized that it was easy to comb.
Δ : Four to seven of ten panelists recognized that it was easy to comb.
× : Only one to three of 10 panelists recognized that it was easy to comb.

Table 1

| | Examples | | | | Comparative Examples | |
|---|---|---|---|---|---|---|
| Polymer | P-1 | P-2 | P-3 | P-4 | Pc-1 | Pc-2 |
| Distribution of molecular weight Mw/Mn | 1.33 | 1.33 | 2.12 | 1.77 | 3.51 | - |
| Young's modulus Mpa | 400 | 392 | 266 | 120 | 10 | 150 |

Table 1 (continued)

| | Examples | | | | Comparative Examples | |
|---|---|---|---|---|---|---|
| Polymer | P-1 | P-2 | P-3 | P-4 | Pc-1 | Pc-2 |
| fracture-point elongation percentage % | 160 | 110 | 155 | 280 | >500 | 3 |
| Curl-retention ratio | ◎ | ◎ | ◎ | ◎ | ○ | × |
| Washability | ○ | ○ | ○ | ○ | × | Δ |
| Styling Ability | ○ | ○ | ○ | ○ | Δ | ○ |
| Style-Keeping Ability | ○ | ○ | ○ | ○ | × | × |
| Finger Touch of Hair | ◎ | ◎ | Δ | ○ | Δ | Δ |
| Tacky Touch of Hair | ◎ | ◎ | ○ | ◎ | Δ | Δ |
| Flaking | ○ | ○ | Δ | ○ | Δ | Δ |
| Gloss | ◎ | ◎ | Δ | ○ | × | × |
| Combing Smoothness | ◎ | ◎ | Δ | ◎ | × | × |

It was found from the results shown in Table 1 that the block copolymers P-1 to 4 were superior to comparative random copolymer Pc-1 and conventional anionic polymer Pc-2 in all performances of hair washability, styling ability, moisture resistance and smooth touch.

2. Example 2: Applications to Various Cosmetic Materials and Evaluation

In the following description, amounts of polymer used for the actual formulations in "% by weight" are expressed on the solid basis.

2-1 Shampoo

A shampoo composition shown below was prepared.

| | (% by weight) |
|---|---|
| Sodium polyoxyethylene lauryl sulfate (3EO adduct) | 16% |
| Lauroyl diethanol amide | 2% |
| block copolymer P-5 | 1.5% |
| Flavoring ingredient | 0.2% |
| Mildewproofing agent | 0.1% |
| Colorant | trace amount |
| Pure water | for balancing |
| Total | 100% |

Use of the composition as a shampoo resulted in smooth combing of the washed hair, excellent luster or gloss of the dried hair, and was successful in giving smooth but volume-raised touch. Repetitive shampooing did not result in adverse effects such as producing tacky touch. It was supposed that similar results would be expected also from the compositions using block copolymers P-1 to 4, and P-6.

2-2 Rinse

A rinse composition below was prepared.

| | (% by weight) |
|---|---|
| Steraryl trimethyl ammonium chloride | 1.5% |
| Cetanol | 2% |

(continued)

| | (% by weight) |
|---|---|
| Block copolymer P-4 | 1.5% |
| Flavoring ingredient | 0.2% |
| Pure water | for balancing |
| Total | 100% |

Use of the composition as a rinse resulted in easy and smooth combing of the rinsed hair, and excellent luster or gloss of the dried hair. Repetitive rinsing did not result in adverse effects such as producing tacky touch. It was supposed that similar results would be expected also from the compositions using block copolymers P-1 to 3, P-5 and P-6.

2-3 Hair Spray

The mother liquor for dilution shown below was put in a spray can and the can was filled with a liquefied petroleum gas, to thereby prepare a hair spray composition.

| Mother liquor for dilution | (% by weight) |
|---|---|
| Block copolymer P-4 | 1% |
| Absolute ethanol | 49% |
| Total | 50% |
| Liquefied petroleum gas (3 kg/cm$^2$G, 20°C) | 50% |

Use of the composition as being sprayed to the hair resulted in an excellent style-keeping performance even with a small content of the polymer, and gave desirable gloss, luster and smooth touch of the hair. It was supposed that similar results would be expected also from the compositions using block copolymers P-1 to 3, P-5 and P-6.

2-4 Hair Spray (Comparative Example)

A hair spray was prepared using comparative random copolymers Pc-1 and Pc-2. Use of a composition having a formulation same as that of 2-3 as being sprayed to the hair resulted in only a poor style-keeping performace due to a small content of the polymer, and failed in giving desirable gloss, luster and smooth touch.

2-5 Foam-Type Aerosol

A foam-type aerosol composition was preprared similarly to as described in 2-3.

| Mother liquor for dilution | (% by weight) |
|---|---|
| Block copolymer P-4 | 2% |
| Polyoxyethylene cetyl ether (10 EO adduct) | 0.3% |
| Polyoxyethylene cetyl ether (2 EO adduct) | 0.1% |
| Pure water | 85.6% |
| Total | 88% |
| Liquefied petroleum gas (3 kg/cm$^2$G, 20°C) | 12% |

The composition applied to the hair gave excellent results similarly to as described in 2-3 in the above. It was supposed that similar results would be expected also from the compositions using block copolymers P-1 to 3, P-5 and P-6.

2-6 Styling Lotion

A styling lotion composition shown below was prepared.

| | (% by weight) |
|---|---|
| Block copolymer P-4 | 3% |
| Pure water | 60% |
| Absolute ethanol | 37% |
| Total | 100% |

The composition applied to the hair gave excellent results similarly to as described in 2-3 in the above. It was supposed that similar results would be expected also from the compositions using block copolymers P-1 to 3, P-5 and P-6.

2-7 Styling Lotion (Comparative Example)

A composition was preprared similarly to as described in 2-6 using comparative random copolymers Pc-1 and Pc-2, but use of the composition as a styling lotion resulted in only a poor style-keeping performance, and failed in giving desirable gloss, luster and smooth touch.

2-8 Gel

A gel composition shown below was prepared.

| | (% by weight) |
|---|---|
| Block copolymer P-6 | 1% |
| Carbopol 940* | 0.5% |
| (adjusted to pH7.5 with aqueous KOH solution) | |
| Pure water | 98.5% |
| Total | 100% |

*product of B.F.Goodrich Chemical Co.

The composition applied as a gel to the hair resulted in an excellent hair-style-keeping performance, and in desirable luster, gloss and smooth touch. Repetitive coating of the gel composition and hair wash did not cause tacky touch, or any adverse effects of incompatible sense due to accumulation. It was supposed that similar results would be expected also from the compositions using block copolymers P-1 to 5.

2-9 Skin Care Cosmetic Material

Skin care cosmetic material 1 was prepared according to the formulation shown in Table 2 below.

Thus obtained skin care cosmetic material 1 was applied to the skin. It was found that the film obtained after drying gave smooth and desirable touch, rather than incompatible sense nor tacky touch. It was supposed that similar results would be expected also from the compositions using block copolymers P-1 to 4, and P-6.

Skin care cosmetic material 2 was prepared similarly to as described in the above except that a 6% ethanol solution of a cationic polymer (trade name: JR-400, product of Union Carbide) was used, in place of block copolymer P-5, according to the formulation shown in Table 2. Application of the skin care cosmetic material 2 on the skin resulted in tacky touch, rather than smooth touch.

Skin care cosmetic material 3 was prepared similarly to as described in the above except that a 6% ethanol solution of an anionic polymer (trade name: Gantretz A425, product of ISP) was used, in place of block copolymer P-5, according to the formulation shown in Table 2. Application of the skin care cosmetic material 3 on the skin resulted in tacky touch, rather than smooth touch, and the resultant film was peeled off only by a several times of rubbing.

Table 2

| | | Skin Cosmetic 1 | Skin Cosmetic 2 | Skin Cosmetic 3 |
|---|---|---|---|---|
| Polymer | Block copolymer P-5 (6 % solution) | 50% | - | - |
| | Cationic polymer (6 % solution) "JR-400" | - | 50% | - |
| | Anionic polymer (6 % solution) "A425" | - | - | 50% |
| Poly ether-modified silicone "SH3771C" | | 0.3% | 0.3% | 0.3% |
| Water | | 49.7% | 49.7% | 49.7% |

2-10 Nail Care Cosmetic Material

The components listed below were dispersed and mixed in a bead mill for one hour, to thereby prepare nail care cosmetic material 1.

| | (% by weight) |
|---|---|
| Block copolymer P-6 | 40.0% (10.0% on solid basis) |
| Isopropanol | 10.0% |
| Red No. 226 | 0.1% |
| Titanium oxide | 4.9% |
| Ethanol | 40.0% (65.0% as total ethanol) |
| Bentonite | 5.0% |
| (trade name: Bentone EW, product of National Lead Company) | |

The composition applied on the nail dried quickly, and the resultant film was less peelable and showed a good film-keeping property. It was supposed that similar results would be expected also from the compositions using block copolymers P-1 to 5.

3. Example 3: Combined Use with Various Polymers and Evaluation

3-1 Combined Use with Anionic Polymer and Evaluation

3-1-1 Pump Spray

Hair cosmetic materials having formulations shown in Table 3 were prepared by general procedures, applied to the hair, and evaluated for their hair styling performance (curl-retention performance), elasticity and volume in the hair styling, flaking, antistatic measures (surface resistance) after being coated on a polypropylene film, styling power (bending strength of hair yarn bundle) and style-keeping performance (strength of hair yarn bundle after fracture) according to the following procedures. As is obvious from the results shown in Table 3, the hair cosmetic materials using block copolymer P-4 had an excellent hair styling performance, successfully kept the curled shape of the hair even under a high-temperature-high-humidity environment, gave elasticity and volume in the hair style, showed no flaking, showed an excellent antistatic property, and had a desirable style-keeping performance by virtue of its elasticity. On the contrary, the hair cosmetic material using comparative polymer Pc-3 resulted in only an insufficient styling performance, failed in providing volume in the hair style, caused flaking, and gave an insufficient antistatic property, all of which were undesirable in terms of performance.

<Methods of Testing and Evaluation>

(1) Styling Performance (Curl-Retention Performance)

A straight hair yarn bundle of 23 cm in length and 2 g in weight was applied with the hair cosmetic material through a dispenser (or sprayed in a form of aerosol), immediately wound around 1.2-cm diameter curling rod, and dried. The hair yarn bundle removed from the curling rod was hung in a thermo-hygrostat chamber conditioned at 30°C, 95% RH, elongation of the hair yarn bundle was measured, and the obtained value was placed in the equation for evaluating curl retention below to thereby determine curl-retention performance (%).

(1) Equation for evaluationg curl retention:

$$\text{Curl-retention performance (\%)} = [(23-L)/(23-L_0)] \times 100$$

(where, $L_0$ is length (cm) of the hair yarn bundle immdiately after being removed from the curling rod, and L is length (cm) of the curled hair 3 hours after).

○ : Curl-retention performance of 71% or more.
Δ : Curl-retention performance of 40% or more and less than 70%.
× : Curl-retention performance of less than 40%.

(2) Elasticity of Hair Yarn

The hair yarn bundle was processed similarly to as described in the style-keeping performance (curl-retention performance) test described in the above, the resultant curled hair yarn bundle was allowed to stand under a thermo-hygrostatic condition of 23°C, 60% RH, and elasticity of the hair yarn when crushed between fingers was evaluated.

○ : Enough stiffness and desirable elasticity.
Δ : Enough stiffness but poor elasticity.
× : No, or almost no elasticity.

(3) Flaking

A straight hair yarn bundle of 23 cm in length and 2 g in weight was applied with the hair cosmetic material through a dispenser (or sprayed in a form of aerosol), immediately flattened with fingers to thereby prepare a flat plate-formed hair yarn bundle, and dried. The hair yarn bundle was then allowed to stand under a condition of 23°C, 60% RH, combed, and the amount of dropped polymer flakes remaining on the hair yarns was observed under a stereo-microscope (×20).

○ : No polymer flake observed, or only slightly observed.
Δ : Polymer flake observed.
× : A large amount of polymer flake observed.

(4) Antistatic Property (Surface Resistivity)

Liquid of the hair cosmetic material listed in Table 3 was prepared (liquid of the hair cosmetic material intended for use in combination with an injection agent (dimethyl ether: DME, liquefied petroleum gas :LPG) is prepared by adding ethanol in the same weight with the injection agent, before being filled into a spray can). The obtained solution was coated on a discharge-treated polypropylene film using a 22-mil bar coater, dried using a hair dryer, allowed to stand under a condition of 23°C, 60% RH, and the surface resistivity was measured using an insulation resistance meter (High Megohm Meter: product of product of Takeda Riken Kogyo K.K.)

○ : Surface resistance of less than $1 \times 10^{10}$
Δ : Surface resistance of $1 \times 10^{10}$ or above but less than $1 \times 10^{12}$
× : Surface resistance of $1 \times 10^{12}$ or above.

(5) Styling Ability Test (Bending Strength of Hair Yarn Bundle)

A hair yarn bundle of 15 cm long was applied with 0.7 g of the individual samples, immediately shaped in 2-cm width, dried, and allowed to stand for one hour in a thermo-hygrostat chamber conditioned at 23°C, 60% RH. The sample was then placed on the supports 65 mm distant from each other and bent at the center thereof under a constant speed, and maximum load was measured and evaluated according to the following criteria.

○: The maximum load was 200 g or above, and the coated hairs were soft to the touch and there was no incompatibility after measurement.
Δ : The maximum load was 100 g or more, and the coated hairs were soft to the touch, but there was a certain level of incompatibility after measurement.
× : The maximum load was less than 100 g, and the coated hairs were not soft to the touch, and there was incompatibility after measurement.

(6) Style-Keeping Ability Test (Hair Yarn Bundle Bending Test)

After the style-keeping performance test, maximum load after the hair yarn bundle was broken was evaluated according to the following criteria.

○: Maximum load was 15 g or more.
Δ : Maximum load was 10 g or more and less than 15 g.
× : Maximum load was less than 10 g.

3-1-2 Aerosol

Hair cosmetic materials (water-containing aerosol) having a formulation shown in Table 4 were prepared by general procedures, and evaluated similarly to as described for 3-1-1. As is obvious from the results shown in Table 4, the hair cosmetic materials using block copolymer P-4 or P-5 had an excellent hair styling performance, successfully kept the curled shape of the hair even under a high-temperature-high-humidity environment, gave elasticity and volume in the hair style, showed no flaking, showed an excellent antistatic property, and had a desirable style-keeping performance by virtue of rubber elasticity of the polymer film. On the contrary, the hair cosmetic materials using comparative polymer Pc-3 resulted in only an insufficient styling performance, failed in providing volume in the hair style, caused flaking, and gave an insufficient antistatic property, all of which were undesirable in terms of performance.

Table 3

(%:Conversion amounts of activate ingredients)

| 3-1-1 Pump Spray | | 1 | 2 | 3 | 4 | 5 | 6 | 7 | 8 | 9 |
|---|---|---|---|---|---|---|---|---|---|---|
| Composition | P-4 | 2.0 | 2.0 | 2.0 | — | — | — | — | — | — |
| | Anionic polymer 1 | 1.0 | — | — | 3.0 | — | — | 1.0 | — | — |
| | Anionic Polymer 2 | — | 1.0 | — | — | 3.0 | — | — | 1.0 | — |
| | Anionic Polymer 3 | — | — | 1.0 | — | — | 3.0 | — | — | 1.0 |
| | Pc-3 | — | — | — | — | — | — | 2.0 | 2.0 | 2.0 |
| | Pure water | 45.0 | 45.0 | 45.0 | 45.0 | 45.0 | 45.0 | 45.0 | 45.0 | 45.0 |
| | Ethanol | balance | balance | balance | balance | balance | balance | balance | balance | balance |
| | Total | 100.0 | 100.0 | 100.0 | 100.0 | 100.0 | 100.0 | 100.0 | 100.0 | 100.0 |
| Evaluation results | (1) Styling performance | ○ | ○ | ○ | △ | × | × | ○ | ○ | ○ |
| | (2)Elasticity of hairs | ○ | ○ | ○ | △ | △ | △ | ○ | ○ | ○ |
| | (3)Flaking | ○ | ○ | ○ | × | × | × | ○ | ○ | ○ |
| | (4)Antistatic property | ○ | ○ | ○ | × | × | × | ○ | ○ | ○ |
| | (5)Styling ability | ○ | ○ | ○ | ○ | ○ | ○ | △ | △ | △ |
| | (6)Style-Keeping ability | ○ | ○ | ○ | × | × | × | △ | × | × |

Table 4

(%:Conversion amounts of activate ingredients)

| 3-1-2 Aerosol | | 1 | 2 | 3 | 4 | 5 | 6 | 7 | 8 |
|---|---|---|---|---|---|---|---|---|---|
| Composition | P-5 | 2.0 | 2.0 | — | — | — | — | — | — |
| | P-4 | 1.0 | — | 2.0 | 2.0 | — | — | — | — |
| | Anionic polymer 1 | — | 1.0 | — | — | 3.0 | — | 1.0 | — |
| | Anionic Polymer 2 | — | — | 1.0 | — | — | 3.0 | — | 1.0 |
| | Pc-3 | — | — | — | 1.0 | — | — | 2.0 | 2.0 |
| | Pure water | 20.0 | 20.0 | 20.0 | 20.0 | 20.0 | 20.0 | 20.0 | 20.0 |
| | Ethanol | balance | balance | balance | balance | balance | balance | balance | balance |
| | Concentrate solution | 60.0 | 60.0 | 60.0 | 60.0 | 60.0 | 60.0 | 60.0 | 60.0 |
| | LPG | 10.0 | 10.0 | 10.0 | 10.0 | 10.0 | 10.0 | 10.0 | 10.0 |
| | DME | 30.0 | 30.0 | 30.0 | 30.0 | 30.0 | 30.0 | 30.0 | 30.0 |
| | Total | 100.0 | 100.0 | 100.0 | 100.0 | 100.0 | 100.0 | 100.0 | 100.0 |
| Evaluation results | (1) Styling performance | ○ | ○ | ○ | ○ | × | × | ○ | ○ |
| | (2) Elasticity of hairs | ○ | ○ | ○ | ○ | △ | △ | ○ | ○ |
| | (3) Flaking | ○ | ○ | ○ | ○ | × | × | ○ | ○ |
| | (4) Antistatic Property | ○ | ○ | ○ | ○ | × | × | ○ | ○ |
| | (5) Styling ability | ○ | ○ | ○ | ○ | ○ | ○ | △ | △ |
| | (6) Style-Keeping ability | ○ | ○ | ○ | ○ | × | × | △ | × |

P-4: Block copolymer prepared in Exemplary Manufacture 4

P-5: Block copolymer prepared in Exemplary Manufacture 5

Pc-3: Amine-oxide-group-containing copolymer prepared in Exemplary Manufacture 8

Anionic polymer 1: Diahold LP503 (product of Mitsubishi Chemical Corporation)

Anionic polymer 2: Partially nuetralized product obtained by neutralizing 20 mol% of acid portion of Gantrez ES-225 (product of ISP) with 2-amino-2-methyl-1-propanol

Anionic polymer 3: Partially nuetralized product obtained by neutralizing 90 mol% of acid portion of Resin 28-2930 (product of National Starch and Chemical Company) with 2-amino-2-methyl-1-propanol.

3-2 Combined Use with Cationic Polymer and Evaluation

3-2-1 Pump Spray

Hair cosmetic materials (pump spray) as being combined with a cationic polymer according to the formulation shown in Table 5 were prepared by general procedures, applied to the hair, and evaluated for their hair styling performance (style-keeping performance), elasticity and tacky touch under high humidity, slipping feel and combing smoothness during coating, and smoothness of the dried hair, according to the following procedures. Results were shown in Table 5.

As is obvious from the results shown in Table 5, the hair cosmetic materials using block copolymer P-4 had an excellent hair styling performance, successfully kept the curled shape of the hair even under a high-temperature-high-humidity environment, gave an desirable elasticity without causing tacky touch, showed a desirable finger touch and combing smoothness during coating, and produced smooth feel of touch of the dried hair yarns. On the contrary, the hair cosmetic materials using comparative polymer Pc-3 or the like resulted in only an insufficient styling performance, produced tacky touch under the high-humidity environment, and showed only a limited elasticity, all of which were undesirable in terms of performance.

<Methods of Testing and Evaluation>

(1) Styling performance (curl-retention performance) and (2) elasticity of hair yarn were evaluated similarly to as described in 3-1-1.

(3) Tacky Touch of Hair

The curled hair yarn bundle obtained similarly to as described for the styling performance (curl-retention performance) test was allowed to stand under a thermo-hygrostat condition of 23°C, 60% RH, and tackiness of the curl

felt by a finger was evaluated.

○ : No tackiness
Δ : Slight tackiness
× : Considerable tackiness

(4) Slipping Feel under Moist Condition (Combing Smoothness)

A straight hair yarn bundle of 23 cm in length and 2 g in weight was coated with a predetermined amount of the individual hair cosmetic samples, immediately touched by fingers, and slipping feel and combing smoothness were evaluated.

○ : Desirable slipping feel, smooth combing
Δ : Poor slipping feel, fairly smooth combing
× : No slipping feel, difficulty in combing

(5) Smoothness of Dried Hair

A straight hair yarn bundle of 23 cm in length and 2 g in weight was coated with a predetermined amount of the individual hair cosmetic samples, and dried using a hair dryer. The dried hair yarn bundle was then allowed to stand under a thermo-hygrostat condition of 23°C, 60% RH, and smoothness of the hair yarn felt by a finger was evaluated.

○ : Smooth touch
Δ : Somewhat poor in the smoothness.
× : Not smooth, and some resistance felt.

3-2-2 Foam-Type Hair Spray

Hair cosmetic materials (foam-type hair spray) as being combined with a cationic polymer according to the formulation shown in Table 6 were prepared by general procedures, and evaluated similarly to as described in 3-2-1. Results were shown in Table 6.

As is obvious from the results shown in Table 6, the hair cosmetic materials using block copolymer P-4 or P-5 had an excellent hair styling performance, successfully kept the curled shape of the hair even under a high-temperature-high-humidity environment, gave an desirable elasticity without causing tacky touch, showed a desirable finger touch and combing smoothness during coating, and produced smooth feel of touch of the dried hair yarns. On the contrary, the hair cosmetic materials using comparative polymer Pc-3 or the like resulted in only an insufficient styling performance, produced tacky touch under the high-humidity environment, and showed only a limited elasticity, all of which were undesirable in terms of performance.

Table 5

(%:Conversion amounts of activate ingredients)

| 3-2-1 Pump spray | | 1 | 2 | 3 | 4 | 5 | 6 | 7 |
|---|---|---|---|---|---|---|---|---|
| Composition | P-4 | 2.5 | 2.0 | 2.0 | — | — | — | — |
| | Cationic polymer 1 | 0.5 | 1.0 | — | 3.0 | — | 1.0 | — |
| | Cationic polymer 2 | — | — | 1.0 | — | 3.0 | — | 1.0 |
| | Pc-3 | — | — | — | — | — | 2.0 | 2.0 |
| | Pure water | 45.0 | 45.0 | 45.0 | 45.0 | 45.0 | 45.0 | 45.0 |
| | Ethanol | balance | balance | balance | balance | balance | balance | balance |
| | Total | 100.0 | 100.0 | 100.0 | 100.0 | 100.0 | 100.0 | 100.0 |
| Evaluation Results | (1)Styling performance | ○ | ○ | ○ | × | × | ○ | ○ |
| | (2)Elasticity of hairs | ○ | ○ | ○ | △ | △ | ○ | ○ |
| | (3)Tacky Touch | ○ | ○ | ○ | × | × | △ | △ |
| | (4)Slipping Feel under moist condition | ○ | ○ | ○ | ○ | △ | ○ | △ |
| | (5)Smoothness | ○ | ○ | ○ | ○ | ○ | △ | △ |

Table 6

(%:Conversion amounts of activate ingredients)

| 3-2-2 Aerosol | | 1 | 2 | 3 | 4 | 5 | 6 | 7 | 8 |
|---|---|---|---|---|---|---|---|---|---|
| Composition | P-5 | 2.0 | 2.0 | — | — | — | — | — | — |
| | P-4 | — | — | 2.0 | 2.0 | — | — | — | — |
| | Cationic Polymer 1 | 1.0 | — | 1.0 | — | 3.0 | — | 1.0 | — |
| | Cationic Polymer 2 | — | 1.0 | — | 1.0 | — | 3.0 | — | 1.0 |
| | Pc-3 | — | — | — | 1.0 | — | — | 2.0 | 2.0 |
| | Nonionic surfactant 3 | 0.5 | 0.5 | 0.5 | 0.5 | 0.5 | 0.5 | 0.5 | 0.5 |
| | Pure water | balance | balance | balance | balance | balance | balance | balance | balance |
| | Concentrate solution | 92.5 | 92.5 | 92.5 | 92.5 | 92.5 | 92.5 | 92.5 | 92.5 |
| | LPG | 7.5 | 7.5 | 7.5 | 7.5 | 7.5 | 7.5 | 7.5 | 7.5 |
| | Total | 100.0 | 100.0 | 100.0 | 100.0 | 100.0 | 100.0 | 100.0 | 100.0 |
| Evaluation results | (1)Styling performance | ○ | ○ | ○ | ○ | × | × | ○ | ○ |
| | (2)Elasticity of hairs | ○ | ○ | ○ | ○ | △ | × | △ | ○ |
| | (3)Tacky Touch | ○ | ○ | ○ | ○ | × | × | ○ | △ |
| | (4)Slipping Feel under moist condition | ○ | ○ | ○ | ○ | ○ | △ | △ | △ |
| | (5)Smoothness | ○ | ○ | ○ | ○ | ○ | ○ | ○ | △ |

P-4: Block copolymer prepared in Exemplary Manufacture 4

P-5: Block copolymer prepared in Exemplary Manufacture 5

Pc-3: Amine-oxide-group-containing polymer prepared in Exemplary Manufacture 8

Cationic polymer 1: Ucare Polymer JR-400 (product of Amacol) (cationized hydroxyethyl cellulose)

Cationic polymer 2: Gafquat 755N (product of ISP) (N-vinylpyrrolidone/quaternized dimethylaminoethyl methacrylate copolymer)

Nonionic surfactant 3: Nikkol BL9-EX (product of Nikko Chemicals Co., Ltd.) (Polyoxyethylene (n=9) lauryl ether)

3-3 Combined Use with Nonionic Polymer and Evaluation

3-3-1 Pump Spray

Hair cosmetic materials (pump spray) as being combined with a nonionic polymer according to the formulation shown in Table 7 were prepared by general procedures, applied to the hair, and evaluated for their hair styling performance (style-keeping performance), elasticity and tacky touch under high humidity, slipping feel and combing smoothness during coating, and smoothness of the dried hair, according to the following procedures. Results were shown in Table 7.

As is obvious from the results shown in Table 7, the hair cosmetic materials using block copolymer P-4 had an excellent hair styling performance, successfully kept the curled shape of the hair even under a high-temperature-high-humidity environment, gave an desirable elasticity without causing tacky touch, showed a desirable finger touch and combing smoothness during coating, and produced smooth feel of touch of the dried hair yarns. On the contrary, the hair cosmetic materials using comparative polymer Pc-3 or the like resulted in only an insufficient styling performance, produced tacky touch under the high-humidity environment, and showed only a limited elasticity, all of which were undesirable in terms of performance.

<Methods of Testing and Evaluation>

(1) Styling performance (curl-retention performance), (2) elasticity of hair yarn, (4) flaking, and (5) antistatic were evaluated similarly to as described in 3-1-1, and (6) slipping Feel under moist condition was evaluated similarly to as described in 3-2-1.

(3) Tacky Touch of Hair under High-Humidity Condition

The hair yarn bundle was processed similarly to as described in the styling performance (curl-keeping performance) test described in the above, the resultant curled hair yarn bundle was allowed to stand overnight under a thermo-hygrostatic condition of 30°C, 90% RH, and tacky touch felt by the fingers was evaluated.

○ : No tackiness
Δ : Slight tackiness

× : Considerable tackiness

3-3-2 Aerosol

Hair cosmetic materials (aerosol) as being combined with a nonionic polymer according to the formulation shown in Table 8 were prepared by general procedures, and evaluated similarly to as described in 3-3-1. Results were shown in Table 8.

As is obvious from the results shown in Table 8, the hair cosmetic materials using block copolymer P-4 or P-5 had an excellent hair styling performance, successfully kept the curled shape of the hair even under a high-temperature-high-humidity environment and gave elasticity and volume in the hair style. It was also found that they allowed smooth combing during coating, caused no flaking, and showed an excellent antistatic property. On the contrary, the hair cosmetic materials using comparative polymer Pc-3 resulted in only an insufficient styling performance, produced tacky touch under a high-temperature-high-humidity condition only to show a poor elasticity, failed in ensuring combing smoothness, caused flaking, and gave only an insufficient antistatic property, all of which were undesirable in terms of performance.

3-3-3 Hair Gel

Hair cosmetic materials (hair gel) as being combined with a nonionic polymer according to the formulation shown in Table 9 were prepared by general procedures, and evaluated similarly to as described in 3-3-1.

As is obvious from the results shown in Table 9, the hair cosmetic materials for hair gel using block copolymer P-4 or P-5 had an excellent hair styling performance, successfully kept the curled shape of the hair even under a high-temperature-high-humidity environment and gave elasticity and volume in the hair style, allowed smooth combing, caused no flaking, and showed an excellent antistatic property.

On the contrary, the hair cosmetic materials using comparative polymer Pc-3 resulted in only an insufficient styling performance, produced tacky touch under a high-temperature-high-humidity condition or failed in providing elasticity to the hair, failed in ensuring combing smoothness, caused flaking, and gave only an insufficient antistatic property, all of which were undesirable in terms of performance.

Table 7

(%:Conversion amounts of activate ingredients)

| 3-3-1 Punm spray | | 1 | 2 | 3 | 4 | 5 | 6 | 7 | 8 |
|---|---|---|---|---|---|---|---|---|---|
| Composition | P-4 | 2.5 | 2.0 | 0.5 | 2.0 | — | — | — | — |
| | Nonionic polymer 1 | 0.5 | 1.0 | 2.5 | — | 3.0 | — | 1.0 | — |
| | Nonionic polymer 2 | — | — | — | 1.0 | — | 3.0 | — | 1.0 |
| | Pc-3 | — | — | — | — | — | — | 2.0 | 2.0 |
| | Pure water | 45.0 | 45.0 | 45.0 | 45.0 | 45.0 | 45.0 | 45.0 | 45.0 |
| | Ethanol | balance | balance | balance | balance | balance | balance | balance | balance |
| | Total | 100.0 | 100.0 | 100.0 | 100.0 | 100.0 | 100.0 | 100.0 | 100.0 |
| Evaluation results | (1)Styling performance | ○ | ○ | ○ | ○ | ×× | ×× | ○ | ○ |
| | (2)Elasticity of hairs | ○ | ○ | ○ | ○ | △ | △ | ○ | ○ |
| | (3)Tacky touch of hair under high-humidity condition | ○ | ○ | ○ | ○ | × | × | △ | △ |
| | (3)Flaking | ○ | ○ | ○ | ○ | × | × | ○ | ○ |
| | (4)Antistatic property | ○ | ○ | ○ | ○ | △ | △ | ○ | ○ |
| | (5)Slipping feel under moist condition | ○ | ○ | ○ | ○ | × | × | △ | △ |

Table 8

(%:Conversion amounts of activate ingredients)

| 3-3-2 Aerosol | | 1 | 2 | 3 | 4 | 5 | 6 | 7 | 8 |
|---|---|---|---|---|---|---|---|---|---|
| Composition | P-5 | 2.0 | 2.0 | — | — | — | — | — | — |
| | P-4 | — | — | 2.0 | 2.0 | — | — | — | — |
| | Nonionic polymer 1 | 1.0 | — | 1.0 | — | 3.0 | — | 1.0 | — |
| | Nonionic polymer 2 | — | 1.0 | — | 1.0 | — | 3.0 | — | 1.0 |
| | Pc-3 | — | — | — | 1.0 | — | — | 2.0 | 2.0 |
| | Pure water | 20.0 | 20.0 | 20.0 | 20.0 | 20.0 | 20.0 | 20.0 | 20.0 |
| | Ethanol | balance | balance | balance | balance | balance | balance | balance | balance |
| | Concentrate solution | 60.0 | 60.0 | 60.0 | 60.0 | 60.0 | 60.0 | 60.0 | 60.0 |
| | LPG | 10.0 | 10.0 | 10.0 | 10.0 | 10.0 | 10.0 | 10.0 | 10.0 |
| | Total | 100.0 | 100.0 | 100.0 | 100.0 | 100.0 | 100.0 | 100.0 | 100.0 |
| Evaluation results | (1)Styling performance | ○ | ○ | ○ | ○ | × | × | ○ | ○ |
| | (2)Elasticity of hairs | ○ | ○ | ○ | ○ | △ | △ | ○ | ○ |
| | (3)Tacky touch of hair under high-humidity condition | ○ | ○ | ○ | ○ | × | × | △ | △ |
| | (3)Flaking | ○ | ○ | ○ | ○ | × | × | ○ | △ |
| | (4)Antistatic Property | ○ | ○ | ○ | ○ | △ | △ | ○ | ○ |
| | (5)slipping feel under moist condition | ○ | ○ | ○ | ○ | × | × | △ | △ |

Table 9

(%:Conversion amounts of activate ingredients)

| 3-3-3 Hair gel | | 1 | 2 | 3 | 4 | 5 | 6 | 7 | 8 |
|---|---|---|---|---|---|---|---|---|---|
| Composition | P-5 | 2.0 | 2.0 | — | — | — | — | — | — |
| | P-4 | — | — | 2.0 | 2.0 | — | — | — | — |
| | Nonionic polymer 1 | 1.0 | — | 1.0 | — | 3.0 | — | 1.0 | — |
| | Nonionic polymer 2 | — | 1.0 | — | 1.0 | — | 3.0 | — | 1.0 |
| | Pc-3 | — | — | — | 1.0 | — | — | 2.0 | 2.0 |
| | Neutralized of carboxyvinyl polymer | 0.5 | 0.5 | 0.5 | 0.5 | 0.5 | 0.5 | 0.5 | 0.5 |
| | Pure water | balance | balance | balance | balance | balance | balance | balance | balance |
| | Total | 100.0 | 100.0 | 100.0 | 100.0 | 100.0 | 100.0 | 100.0 | 100.0 |
| Evaluation results | (1)Styling performance | ○ | ○ | ○ | ○ | × | × | ○ | ○ |
| | (2)Elasticity of hairs | ○ | ○ | ○ | ○ | △ | △ | ○ | ○ |
| | (3)Tacky Touch of Hair under High-Humidity Condition | ○ | ○ | ○ | ○ | × | × | △ | △ |
| | (3)Flaking | ○ | ○ | ○ | ○ | × | × | ○ | △ |
| | (4)Antistatic Property | ○ | ○ | ○ | ○ | △ | △ | ○ | ○ |
| | (5)Slipping feel under moist condition | ○ | ○ | ○ | ○ | × | × | △ | △ |

P-4: Block copolymer prepared in Exemplary Manufacture 4

P-5: Block copolymer prepared in Exemplary Manufacture 5

Pc-3: Amine-oxide-group-containing copolymer prepared in Exemplary Manufacture 8

Nonionic polymer 1: Luviskol VA64 (product of BASF AG) (vinylpyrrolidone/vinyl acetate copolymer)

Nonionic polymer 2: Luviskol K90 (product of BASF AG) (polyvinylpyrrolidone)

Neutralized carboxyvinyl polymer: Neutralized product of Carbopol 940 (product of B.F. Goodrich Chemical Co.) obtained by neutralizing 70 mol% of acid portion thereof with 2-amino-2-methyl-1-propanol

3-4 Combined Use with Amphoteric Polymer and Evaluation

3-4-1 Pump Spray

Hair cosmetic materials (pump spray) as being combined with an amphoteric polymer according to the formulation shown in Table 10 were prepared by general procedures, applied to the hair, and evaluated for their hair styling performance (style-keeping performance), elasticity under high humidity, volume and tension of the hair, flaking, and washability, according to the following procedures. Results were shown in Table 10.

As is obvious from the results shown in Table 10, the hair cosmetic materials using block copolymer P-4 had an excellent hair styling performance and style-keeping performance, successfully kept the curled shape of the hair even under a high-temperature-high-humidity environment, gave elasticity and volume in the hair style, caused no flaking, and showed an excellent washability.

On the contrary, the hair cosmetic materials using comparative polymer Pc-3 resulted in only an insufficient styling performance and style-keeping performance, failed in providing volume to the hair, caused flaking, and gave only an insufficient washability, all of which were undesirable in terms of performance.

<Methods of Testing and Evaluation>

(1) Styling performance (curl-retention performance), (2) elasticity of hair yarn, (3) flaking, (5) styling ability (bending strength of hair yarn) and (6) style-keeping ability test (hair yarn bundle bending test) were evaluated similarly to as described in 3-1-1.

(4) Washability (Film Solubility)

The prepared hair cosmetic material (excluding an injection agent for any material to be used with the injection agent) was coated on a glass plate using a 100 μm applicator, and dried. The glass plate was then allowed to stand overnight under a thermo-hygrostatic condition of 23°C, 60% RH, dipped into warm water of 40°C, and time required for solubilizing the polymer film formed on the glass plate was measured.

○: The polymer film was solubilized within one minute.
Δ : The polymer film was solubilized in 1 to 5 minutes.

× : Solubilization of the polymer film took 5 minutes or longer.

3-4-2 Aerosol

Hair cosmetic materials (aerosol) as being combined with an amphoteric polymer according to the formulation shown in Table 11 were prepared by general procedures, and evaluated similarly to as described in 3-4-1. Results were shown in Table 11.

As is obvious from the results shown in Table 11, the hair cosmetic materials using block copolymer P-4 or P-5 had an excellent hair styling performance and style-keeping performance, successfully kept the curled shape of the hair even under a high-temperature-high-humidity environment, caused no flaking, and showed an excellent washability. On the contrary, the hair cosmetic materials using comparative polymer Pc-3 resulted in only an insufficient styling performance and style-keeping performance, failed in providing volume to the hair, caused flaking, and gave only an insufficient washability, all of which were undesirable in terms of performance.

Table 10

(%:Conversion amounts of activate ingredients)

| 3-4-1 Punm spray | | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| Compostion | P-4 | 2.5 | 2.0 | 0.5 | 2.0 | 2.0 | 2.0 | – | – | – | – | – | – | – | – |
| | Amphoteric polymer 1 | 0.5 | 1.0 | 2.5 | – | – | – | 3.0 | – | – | – | 1.0 | – | – | – |
| | Amphoteric polymer 2 | – | – | – | 1.0 | – | – | – | 3.0 | – | – | – | 1.0 | – | – |
| | Amphoteric polymer 3 | – | – | – | – | 1.0 | – | – | – | 3.0 | – | – | – | 1.0 | – |
| | Amphoteric polymer 4 | – | – | – | – | – | 1.0 | – | – | – | 3.0 | – | – | – | 1.0 |
| | Pc-3 | – | – | – | – | – | – | – | – | – | – | 2.0 | 2.0 | 2.0 | 2.0 |
| | Pure water | 45.0 | 45.0 | 45.0 | 45.0 | 45.0 | 45.0 | 45.0 | 45.0 | 45.0 | 45.0 | 45.0 | 45.0 | 45.0 | 45.0 |
| | Ethanol | balance | balance | balance | balance | balance | balance | balance | balance | balance | balance | balance | balance | balance | balance |
| | Total | 100.0 | 100.0 | 100.0 | 100.0 | 100.0 | 100.0 | 100.0 | 100.0 | 100.0 | 100.0 | 100.0 | 100.0 | 100.0 | 100.0 |
| Evaluation results | (1)Styling performance | ○ | ○ | ○ | ○ | ○ | ○ | ○ | ○ | ○ | × | ○ | ○ | ○ | ○ |
| | (2)Elasticity of hairs | ○ | ○ | ○ | ○ | ○ | ○ | △ | △ | △ | △ | △ | ○ | △ | △ |
| | (3)Flaking | ○ | ○ | ○ | ○ | ○ | ○ | ○ | ○ | ○ | ○ | ○ | ○ | △ | ○ |
| | (4)Washability | ○ | ○ | ○ | ○ | ○ | ○ | × | × | × | ○ | △ | △ | ○ | ○ |
| | (5)Styling ability | ○ | ○ | ○ | ○ | ○ | ○ | △ | △ | × | × | △ | △ | △ | △ |
| | (6)Style-Keeping ability | ○ | ○ | ○ | ○ | ○ | ○ | △ | △ | × | × | △ | △ | △ | △ |

Table 11

(%:Conversion amounts of activate ingredients)

| 3-4-2 Aerosol | | 1 | 2 | 3 | 4 | 5 | 6 | 7 | 8 |
|---|---|---|---|---|---|---|---|---|---|
| Composition | P-5 | 2.0 | 2.0 | — | — | — | — | — | — |
| | P-4 | — | — | 2.0 | 2.0 | — | — | — | — |
| | Amphoteric polymer 1 | 1.0 | — | 1.0 | — | 3.0 | — | 1.0 | — |
| | Amphoteric polymer 3 | — | 1.0 | — | 1.0 | — | 3.0 | — | 1.0 |
| | Pc-3 | — | — | — | 1.0 | — | — | 2.0 | 2.0 |
| | Pure water | 20.0 | 20.0 | 20.0 | 20.0 | 20.0 | 20.0 | 20.0 | 20.0 |
| | Ethanol | balance | balance | balance | balance | balance | balance | balance | balance |
| | Concentrate solution | 60.0 | 60.0 | 60.0 | 60.0 | 60.0 | 60.0 | 60.0 | 60.0 |
| | LPG | 10.0 | 10.0 | 10.0 | 10.0 | 10.0 | 10.0 | 10.0 | 10.0 |
| | DME | 30.0 | 30.0 | 30.0 | 30.0 | 30.0 | 30.0 | 30.0 | 30.0 |
| | Total | 100.0 | 100.0 | 100.0 | 100.0 | 100.0 | 100.0 | 100.0 | 100.0 |
| Evaluation results | (1)Styling performance | ○ | ○ | ○ | ○ | ○ | △ | ○ | ○ |
| | (2)Elasticity of hairs | ○ | ○ | ○ | ○ | △ | △ | △ | △ |
| | (3)Flaking | ○ | ○ | ○ | ○ | ○ | ○ | ○ | △ |
| | (4)Washability | ○ | ○ | ○ | ○ | × | ○ | △ | ○ |
| | (5)Styling ability | ○ | ○ | ○ | ○ | △ | △ | △ | △ |
| | (6)Style-Keeping ability | ○ | ○ | ○ | ○ | × | × | △ | △ |

P-4: Block copolymer prepared in Exemplary Manufacture 4
P-5: Block copolymer prepared in Exemplary Manufacture 5
Pc-3: Amine-oxide-group-containing copolymer prepared in Exemplary Manufacture 8
Amphoteric polymer 1: Yukaformer Amphoset (product of Mitsubishi Chemical Corporation) (methacryloyl carboxybetaine/alkyl methacrylate copolymer)
Amphoteric polymer 2: Yukaformer SM (product of Mitsubishi Chemical Corporation) (methacryloyl carboxybetaine/alkyl methacrylate copolymer)
Amphoteric polymer 3: Amphomer 28-4910 (product of National Starch and Chemical Company) (octylamido acrylate/butylaminoethyl methacrylate/acrylic acid copolymer) (100% of acid equivalence neutralized with 2-amino-2-methyl-1-propanol)
Amphoteric polymer 4: MarcoatPlus 3330 (product of Cargon) (diallyl dimethyl ammonium chloride/ acrylic acid/acrylamide copolymer)

3-5 Combined Use with Amine-Oxide-Group-Containing Polymer and Evaluation

3-5-1 Pump Spray

Hair cosmetic materials (pump spray) having the formulation shown in Table 12 were prepared by general procedures, applied to the hair, and evaluated for their hair styling performance (style-keeping performance), volume and tension of the hair, flaking, and washability, according to the following procedures. Results were shown in Table 12.
As is obvious from the results shown in Table 12, the hair cosmetic materials using block copolymer P-4 had an excellent hair styling performance, successfully kept the curled shape of the hair even under a high-temperature-high-humidity environment, gave elasticity and volume in the hair style, caused no flaking, and showed an excellent washability.
On the contrary, the hair cosmetic materials using comparative polymer Pc-3 or Pc-4 resulted in only an insufficient styling performance, failed in providing volume to the hair, caused flaking, and gave only an insufficient washability, all of which were undesirable in terms of performance.

<Methods of Testing and Evaluation>

(1) Styling performance (curl-retention performance), (2) elasticity of hair yarn, (3) flaking, (5) styling ability

(bending strength of hair yarn) and (6) style-keeping ability test (hair yarn bundle bending test) were evaluated similarly to as described in 3-1-1. (4) Washability (film solubility) was evaluated similarly to as described in 3-4-1.

3-5-2 Aerosol

Hair cosmetic materials (water-containing aerosol) having the formulation shown in Table 13 were prepared by general procedures, and evaluated similarly to as described in 3-5-1. As is obvious from the results shown in Table 13, the hair cosmetic materials using block copolymer P-4 or P-5 had an excellent hair styling performance, successfully kept the curled shape of the hair even under a high-temperature-high-humidity environment, caused no flaking, and showed an excellent washability.

On the contrary, the hair cosmetic materials using comparative polymer Pc-3 or Pc-4 resulted in only an insufficient styling performance and style-keeping performance, failed in providing volume to the hair, caused flaking, and gave only an insufficient washability, all of which were undesirable in terms of performance.

Table 12

(%:Conversion amounts of activate ingredients)

| 3-5-1 | Pump spray | 1 | 2 | 3 | 4 | 5 | 6 |
|---|---|---|---|---|---|---|---|
| Composition | P-4 | 2.0 | 2.0 | 2.0 | 3.0 | — | — |
| | Pc-3 | 1.0 | — | 2.0 | — | 3.0 | — |
| | Pc-4 | — | 1.0 | — | — | — | 3.0 |
| | Pure water | 45.0 | 45.0 | 45.0 | 45.0 | 45.0 | 45.0 |
| | Ethanol | balance | balance | balance | balance | balance | balance |
| | Total | 100.0 | 100.0 | 100.0 | 100.0 | 100.0 | 100.0 |
| Evaluation results | (1) Styling performance | ○ | ○ | ○ | ○ | △ | × |
| | (2) Elasticity of hairs | ○ | ○ | ○ | △ | △ | △ |
| | (3) Flaking | ○ | ○ | ○ | ○ | × | × |
| | (4) Washability | ○ | ○ | ○ | ○ | ○ | ○ |
| | (5) Styling ability | ○ | ○ | ○ | ○ | ○ | △ |
| | (6) Style-Keeping ability | ○ | ○ | ○ | ○ | △ | △ |

Table 13

(%:Conversion amounts of activate ingredients)

| 3-5-2 Aerosol | | 1 | 2 | 3 | 4 | 5 | 6 | 7 | 8 |
|---|---|---|---|---|---|---|---|---|---|
| Composition | P-5 | 2.0 | 2.0 | — | — | 3.0 | — | — | — |
| | P-4 | — | — | 2.0 | 2.0 | — | 3.0 | — | — |
| | Pc-3 | 1.0 | — | 1.0 | — | — | — | 3.0 | — |
| | Pc-4 | — | 1.0 | — | 1.0 | — | — | — | 3.0 |
| | Pure water | 20.0 | 20.0 | 20.0 | 20.0 | 20.0 | 20.0 | 20.0 | 20.0 |
| | Ethanol | balance | balance | balance | balance | balance | balance | balance | balance |
| | Concentrate solution | 60.0 | 60.0 | 60.0 | 60.0 | 60.0 | 60.0 | 60.0 | 60.0 |
| | LPG | 10.0 | 10.0 | 10.0 | 10.0 | 10.0 | 10.0 | 10.0 | 10.0 |
| | DME | 30.0 | 30.0 | 30.0 | 30.0 | 30.0 | 30.0 | 30.0 | 30.0 |
| | Total | 100.0 | 100.0 | 100.0 | 100.0 | 100.0 | 100.0 | 100.0 | 100.0 |
| Evaluation results | (1)Styling performance | ○ | ○ | ○ | ○ | ○ | ○ | △ | × |
| | (2)Elasticity of hairs | ○ | ○ | ○ | ○ | △ | △ | △ | △ |
| | (3)Flaking | ○ | ○ | ○ | ○ | ○ | ○ | × | × |
| | (4)Antistatic property | ○ | ○ | ○ | ○ | ○ | ○ | ○ | ○ |
| | (5)Styling ability | ○ | ○ | ○ | ○ | ○ | ○ | ○ | △ |
| | (6)Style-Keeping ability | ○ | ○ | ○ | ○ | ○ | ○ | × | △ |

P-4: Block copolymer prepared in Exemplary Manufacture 4

P-5: Block copolymer prepared in Exemplary Manufacture 5

Pc-3: Amine-oxide-containing copolymer prepared in Exemplary Manufacture 8

Pc-4: Amine-oxide-containing copolymer prepared in Exemplary Manufacture 9

3-6 Combined Use with Silicon Derivative and Evaluation

Various hair cosmetic materials were prepared using the block copolymers and comparative polymers according to the formulations shown below (adjusted to 100% in total).

3-6-1 Shampoo Composition

| | |
|---|---|
| Sodium polyoxyethylene lauryl sulfate (3EO adduct) | 20% |
| Lauroyl diethanolamide | 2.0% |
| Block copolymer P-4 | 2.0% |
| Polyethersilicone derivative (trade name: Toray Silicone SH3771) | 0.5% |
| Flavoring ingredient | 0.2% |
| Preservative | 0.1% |
| Colorant | trace amount |
| Pure water | for balancing |

3-6-2 Rinse Composition

| | |
|---|---|
| Stearyl trimethyl ammonium chloride | 2.0% |
| Cetanol | 2.0% |
| Block copolymer P-5 | 1.5% |
| Dimethyl polysiloxane (trade name: Toray Silicone SH200 200CS) | 5.0% |
| Flavoring ingredient | 0.2% |
| Pure water | for balancing |

3-6-3 Hair Spray Composition

| | |
|---|---|
| Block copolymer P-4 | 1% |
| Polyethersilicone derivative (trade name: Toray Silicone SH3771) | 0.5% |
| Amino-modified silicone derivative (trade name: Toray Silicone SM8702C) | 0.5% |

(continued)

| | |
|---|---|
| Absolute ethanol | 48% |
| Liquefied petroleum gas (3 kg/$cm^2$G, 20°C) | 50% |

3-6-4 Foam-Type Aerosol Composition

| | |
|---|---|
| Block copolymer P-4 | 2% |
| Carboxybetaine-type amphoteric polymer (trade name: Yukaformer AM-75R 205S, product of Mitsubishi Chemical Corporation) | 2% |
| Polyethersilicone derivative (trade name: Toray Silicone SH3771) | 1.0% |
| Poly dimethylsiloxane (trade name: Toray Silicone BY11-007) | 1.0% |
| Polyoxyethylene cetyl ether (10EO adduct) | 0.3% |
| Polyoxyethylene cetyl ether (2EO adduct) | 0.1% |
| Pure water | 81.6% |
| Liquefied petroleum gas (3kg/$cm^2$G, 20°C) | 12% |

3-6-5 Styling Lotion Composition

| | |
|---|---|
| Block copolymer P-4 | 1% |
| Polyethersilicone derivative (trade name: Toray Silicone SH3771) | 0.2% |
| Pure water | 60% |
| Absolute ethanol | 38.8% |

3-6-6 Gel Composition

| | |
|---|---|
| Block copolymer P-4 | 3% |
| Polyethersilicone derivative (trade name: Toray Silicone SH3771) | 0.05% |
| Gel base (trade name: Carbopol 940, product of B.F. Goodrich Chemical) | 0.5% |
| Pure water | 96.45% |

3-6-7 Shampoo Composition

| | |
|---|---|
| Sodium polyoxyethylene lauryl sulfate (3EO adduct) | 20% |
| Lauloyl diethanol amide | 2.0% |
| Comparative polymer Pc-3 | 2.0% |
| Polyethersilicone derivative (trade name: Toray Silicone SH3771) | 0.5% |
| Flavoring ingredient | 0.2% |
| Preservative | 0.1% |
| Colorant | trace amount |

(continued)

| | |
|---|---|
| Pure water | 75.2% |

3-6-8 Shampoo Composition

| | |
|---|---|
| Sodium polyoxyethylene lauryl sulfate (3EO adduct) | 20% |
| Lauroyl diethanol amide | 2.0% |
| Block copolymer P-4 | 2.0% |
| Flavoring ingredient | 0.2% |
| Preservative | 0.1% |
| Colorant | trace amount |
| Pure water | 75.7% |

3-6-9 Rinse Composition

| | |
|---|---|
| Stearyl trimethylammonium chloride | 2.0% |
| Cetanol | 2.0% |
| Comparative polymer Pc-3 | 1.5% |
| Dimethyl polysiloxane (trade name: Toray Silicone SH200 200CS) | 5.0% |
| Flavoring ingredient | 0.2% |
| Pure water | 89.3% |

3-6-10 Rinse Composition

| | |
|---|---|
| Stearyl trimethyl ammonium chloride | 2.0% |
| Cetanol | 2.0% |
| block copolymer P-5 | 1.5% |
| Flavoring ingredient | 0.2% |
| Pure water | 94.3% |

3-6-11 Hair Spray Composition

| | |
|---|---|
| Comparative polymer Pc-3 | 1.0% |
| Polyethersilicone derivative (trade name: Toray Silicone SH3771) | 0.5% |
| Amino-modified silicone derivative (trade name:Toray Silicone SM8702C) | 0.5% |
| Absolute ethanol | 48% |
| Liquefied petroleum gas (3kg/cm$^2$G, 20°C) | 50% |

3-6-12 Hair Spray Composition

| | |
|---|---|
| Block copolymer P-4 | 1% |
| Absolute ethanol | 49% |
| Liquefied petroleum gas (3kg/$cm^2$G, 20°C) | 50% |

3-6-13 Foam-Type Aerosol Composition

| | |
|---|---|
| Comparative polymer Pc-3 | 2.0% |
| Carboxybetaine-type amphoteric polymer | 2.0% |
| (trade name: Yukaformer Am-75R 205S, product of ofMitsubishi Chemical Corporation) | 2.0% |
| Polyethersilicone derivative | 1.0% |
| (trade name: Toray Silicone SH3771) | |
| Polymer dimethyl polysiloxane | 1.0% |
| (trade name: Toray Silicone BY11-007) | |
| Polyoxyethylene cetyl ether (10EO adduct) | 0.3% |
| Polyoxyethylene cetyl ether (2EO adduct) | 0.1% |
| Pure water | 81.6% |
| Liquefied petroleum gas (3 kg/$cm^2$G, 20°C) | 12% |

3-6-14 Foam-Type Aerosol Composition

| | |
|---|---|
| Block copolymer P-4 | 2.0% |
| Carboxybetaine-type amphoteric polymer | 2.0% |
| (trade name: Yukaformer AM-75R 205S, product of Mitsubishi Chemical Corporation) | |
| Polyoxyethylene cetyl ether (10EO adduct) | 0.3% |
| Polyoxyethylene cetyl ether (2EO adduct) | 0.1% |
| Pure water | 83.6% |
| Liquefied petroleum gas (3 kg/$cm^2$G, 20°C) | 12% |

3-6-15 Styling Lotion Composition

| | |
|---|---|
| Comparative polymerPc-3 | 1.0% |
| Polyethersilicone derivative | 0.2% |
| (trade name: Toray Silicone SH3771) | |
| Pure water | 60% |
| Absolute ethanol | 38.8% |

3-6-16 Styling Lotion Composition

| | |
|---|---|
| Block copolymer P-4 | 1% |
| Pure water | 60% |
| Absolute ethanol | 39% |

3-6-17 Gel Composition

| | |
|---|---|
| Comparative polymer Pc-3 | 3% |
| Polyethersilicone derivative (trade name: Toray Silicone SH3771) | 0.05% |
| Gel base (trade name: Carbopol 940) (product of B.F.Goodrich Chemical) | 0.5% |
| Pure water | 96.45% |

3-6-18 Gel Composition

| | |
|---|---|
| Block copolymer P-4 | 3% |
| Gel base (trade name: Carbopol 940) (product of B.F.Goodrich Chemical, adjusted to pH7.5 with aqueous KOH solution) | 0.5% |
| Pure water | 98.5% |

<Tests and Evaluation>

Using the hair cosmetic materials 3-6-1 to 3-6-18 based on the above formulations as samples, styling ability, tacky touch, smoothness, combing smoothness and hair gloss were evaluated. Methods for the evaluation are as individually described below. Evaluation results were listed in Table 14 for conditioning application, and in Table 15 for styling application.

(Evaluation of Conditioning Application)

(1) Tacky touch

To a bundle of hair yarns (2 g, 23 cm), 1.0 g of the sample was coated, the bundle was then rinsed with a running water for 2 minutes, shaped with a comb, allowed to stand at 23°C, 60%RH for 24 hours, and tacky touch of the bundle was evaluated by sensory inspection.

○ : No tackiness
Δ : Slight tackiness
× : Considerable tackiness

(2) Smoothness

Smoothness of the hair yarn bundle prepared similarly to as described in the above was evaluated by sensory inspection.

○ : Smooth
Δ : Fairly smooth
× : Not smooth

(3) Combing Smoothness

Combing smoothness of the hair yarn bundle prepared similarly to as described in the above was evaluated.

○ : Smooth combing
Δ : Slightly resistive
× : Resistive

(4) Hair gloss

Hair gloss of the hair yarn bundle prepared similarly to as described in the above was evaluated by sensory inspection.

○: Glossy
Δ: Slightly glossy
×: Not gloss

(Evaluation of Styling Application)

(5) Styling ability

A hair yarn bundle of 23 cm in length and 2.0 g in weight was coated with 0.7 g of the individual hair cosmetic materials, wound around a 1-cm diameter rod, allowed to dry, removed from the rod, to thereby obtained a curled hair yarn bundle.

The curled hair yarn bundle was vertically hung for 30 minutes in a thermo-hygrostat chamber preliminarily conditioned at 30°C, 90% RH over 3 hours or more, and the styling ability was evaluated based on degree of decurling of the hair yarn bundle.

○ : Almost no change
Δ : Slight decurling
× : Apparent decurling

(6) Tacky touch

To a bundle of hair yarns (2 g, 23 cm), 0.7 g of the sample was coated, shaped with a comb, allowed to stand at 23°C, 60%RH for 24 hours, and tacky touch of the bundle was evaluated by sensory inspection.

○ : No tackiness
Δ : Slight tackiness
× : Considerable tackiness

(7) Smoothness

Smoothness of the hair yarn bundle prepared similarly to as described in the above was evaluated by sensory inspection.

○ : Smooth
Δ : Fairly smooth
× : Not smooth

(8) Combing Smoothness

Combing smoothness of the hair yarn bundle prepared similarly to as described in the above was evaluated.

○ : Smooth combing
Δ : Slightly resistive
× : Resistive

(9) Hair gloss

Hair gloss of the hair yarn bundle prepared similarly to as described in the above was evaluated by sensory inspection.

○: Glossy
Δ: Slightly glossy
×: Not glossy

Table 14

| (Evaluation results of Conditioning Application) | | | | | |
|---|---|---|---|---|---|
| Hair cosmetic No. | | (1)Tacky touch | (2)Smoothness | (3)Combing Smoothness | (4)Hair gloss |
| 3-6- | 1 | ○ | ○ | ○ | ○ |
| | 2 | ○ | ○ | ○ | ○ |
| | 7 | ○ | Δ | Δ | Δ |
| | 8 | ○ | Δ | Δ | ○ |
| | 9 | ○ | Δ | Δ | Δ |
| | 10 | ○ | × | Δ | ○ |

Table 15

| (Evaluation results of Styling Application) | | | | | | |
|---|---|---|---|---|---|---|
| Hair cosmetic No. | | (5)Styling ability | (6)Tacky touch | (7) Smoothness | (8)Combing Smoothness | (9)Hair gloss |
| 3-6- | 3 | ○ | ○ | ○ | ○ | ○ |
| | 4 | ○ | ○ | ○ | ○ | ○ |
| | 5 | ○ | ○ | ○ | ○ | ○ |
| | 6 | ○ | ○ | ○ | ○ | ○ |
| | 11 | ○ | Δ | Δ | ○ | ○ |
| | 12 | ○ | ○ | Δ | Δ | Δ |
| | 13 | ○ | Δ | Δ | ○ | ○ |
| | 14 | ○ | Δ | Δ | Δ | Δ |
| | 15 | ○ | ○ | Δ | ○ | ○ |
| | 16 | ○ | ○ | Δ | Δ | Δ |
| | 17 | ○ | Δ | Δ | ○ | ○ |
| | 18 | ○ | Δ | Δ | Δ | Δ |

INDUSTRIAL APPLICABILITY

The present invention can provide a cosmetic polymer compoistion and a cosmetic material capable of satisfying a plurality of performances required for the cosmetic material at the same time by using a polymer having two or more properties.

## Claims

1. A cosmetic polymer composition comprising a straight-chain block copolymer having a unit derived from a compound having an ethylenic unsaturated bond, having a number-average molecular weight of $1.0\times10^3$ to $1.0\times10^6$, and having two or more glass transition points or melting points.

2. The cosmetic polymer composition of claim 1, wherein the block copolymer comprises at least one block composed of a unit having a hydrophilic group.

3. The cosmetic polymer composition of claim 2, wherein the hydrophilic group is at least any one selected from groups consisting of an anionic group consisting of carboxylic acid group, sulfonic acid group, phosphonic acid group and salts of these groups; a cationic group consisting of amino group (including quaternary ammonium salt

group), pyridyl group and salts of these groups; a nonionic group consisting of hydroxyl group, alkoxy group, epoxy group, amido group and cyano group; an amphoteric ionic group consisting of carboxybetaine group; and a semipolar group consisting of amine oxide group.

**4.** The cosmetic polymer composition of any one of claims 1 to 3, wherein the block copolymer comprises at least one of units represented by formulae (1) to (5) below:

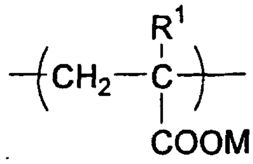

(1)

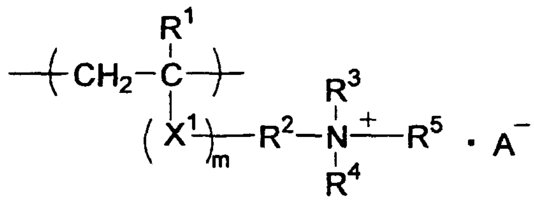

(2)

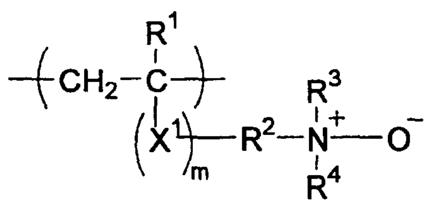

(3)

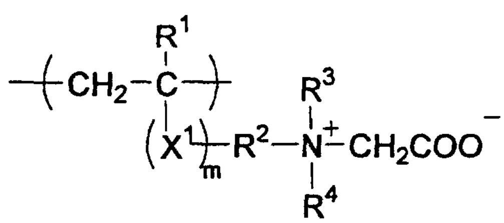

(4)

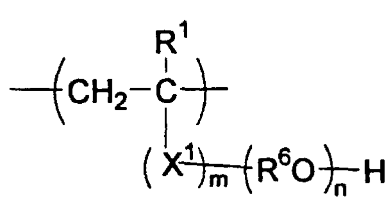

(5)

(where, $R^1$ represents a hydrogen atom or a methyl group; $R^2$ and $R^6$ respectively represent a $C_{1-4}$ straight-chain or branched-chain alkylene group; $R^3$, $R^4$ and $R^5$ respectively represent a hydrogen atom, $C_{1-24}$ alkyl group, $C_{6-24}$ aryl group, or any combination thereof such as $C_{7-24}$ arylalkyl group or alkylaryl group; and $X^1$ represents -COO-, -CONH-, -O- or NH-. $A^-$ represents an anion; and M represents a hydrogen atom, an alkali metal ion or an ammonium ion. m is 0 or 1; and n is any integer from 1 to 50.)

**5.** The cosmetic polymer composition of any one of claims 1 to 4, wherein the block copolymer comprises a unit derived from an ethylenic unsaturated carboxylic acid, and a unit derived from an ethylenic unsaturated carboxylate

ester.

**6.** The cosmetic polymer composition of any one of claims 1 to 5, wherein the block copolymer comprises at least one block formed by post-treatment after polymerization.

**7.** The cosmetic polymer composition of any one of claims 1 to 6, wherein the block copolymer has a glass transition point or a melting point nearly equal to a glass transition point or a melting point of a homopolymer composed of the monomer which make up at least one block of the block copolymer.

**8.** The cosmetic polymer composition of any one of claims 1 to 7, wherein the block copolymer has a ratio (Mw/Mn), which is a ratio of weight-average molecular weight (Mw) to number-average molecular weight (Mn), of 2.5 or less.

**9.** The cosmetic polymer composition of any one of claims 1 to 8, wherein the block copolymer is dispersible or soluble in water and/or alcohol.

**10.** The cosmetic polymer composition of any one of claims 1 to 9, wherein the block copolymer is produced by controlled radical polymerization using an organic halide as an initiator, and using, as a catalyst, at least a metal complex having a metal selected from Group VIII, Group IX, Group X and Group XI elements in the periodic table as a central metal.

**11.** A hair cosmetic polymer composition comprising a copolymer capable of forming a film having a Young's modulus (measured according to JIS K7161 under a tensile speed of 20 mm/min) of 50 MPa or larger and a fracture-point elongation of 100% or larger, and dispersible into water and/or alcohol.

**12.** The hair cosmetic polymer composition of any one of claims 1 to 11, comprising, in addition to the copolymer (a), an anionic polymer (b1) in a ratio by weight ((a)/(b1)) of 1/10 to 10/1.

**13.** The hair cosmetic polymer composition of claim 12, wherein the anionic polymer (b1) is a polymer having an anionic group selected from carboxyl group, sulfonic acid group, phosphonic acid group and salts of these groups.

**14.** The hair cosmetic polymer composition of any one of claims 1 to 11, comprising, in addition to the copolymer (a), a cationic polymer (b2) in a ratio by weight ((a)/(b2)) of 1/10 to 10/1.

**15.** The hair cosmetic polymer composition of claim 14, wherein the cationic polymer (b2) is at least any one cationic polymer selected from ① to ④ below:

① copolymer of which constituents are N-vinylpyrrolidone and/or N-vinylcaprolactam and a cationic-group-containing monomer;
② polymer or copolymer of dimethyl diallyl ammonium;
③ polymer or copolymer of acrylic ester or methacrylic ester quaternary ammonium salt; and
④ quaternary ammonium salt of cellulose-base or chitosan-base polymer.

**16.** The hair cosmetic polymer composition of any one of claims 1 to 11, comprising, in addition to the copolymer (a), a nonionic polymer (b3) in a ratio by weight ((a)/(b3)) of 1/10 to 10/1.

**17.** The hair cosmetic polymer composition of claim 16, wherein the nonionic polymer (b3) is a polymer containing, as a constituent, an unsaturated monomer having at least one functional group selected from pyrrolidone group, amido group (containing N-alkyl amido), polyether group, formamide group and acetamide group.

**18.** The hair cosmetic polymer composition of any one of claims 1 to 11, comprising, in addition to the copolymer (a), an amphoteric polymer (b4) in a ratio by weight ((a)/(b4)) of 1/10 to 10/1.

**19.** The hair cosmetic polymer composition of claim 18, wherein the amphoteric polymer (b4) is a polymer containing, as a constituent thereof, an unsaturated monomer having at least one betaine-structured group such as carboxybetaine group, sulfobetaine group, phosphobetaine group and so forth.

**20.** The hair cosmetic polymer composition of any one of claims 1 to 11, comprising, in addition to the copolymer (a), an amine-oxide-group-containing polymer (b5) in a ratio by weight ((a)/(b5)) of 1/10 to 10/1.

**21.** The hair cosmetic polymer composition of claim 20, wherein the amine-oxide-group-containing polymer comprises a unit derived from amine-oxide-group-containing unsaturated monomer and a unit derived from ethylenic unsaturated carboxylic acid ester, and the amine-oxide-group-containing unsaturated monomer is a compound represented by any one of formulae (b5-1) to (b5-4) below:

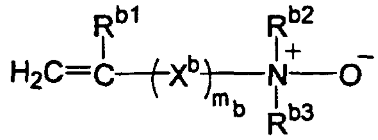

(b5-1)

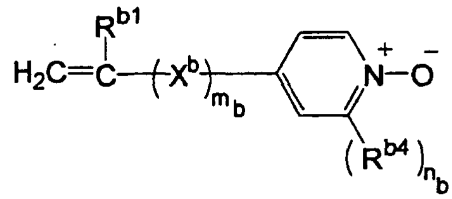

(b5-2)

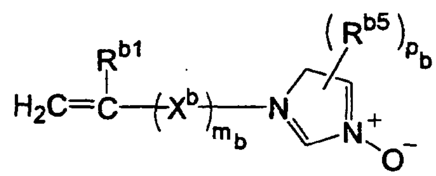

(b5-3)

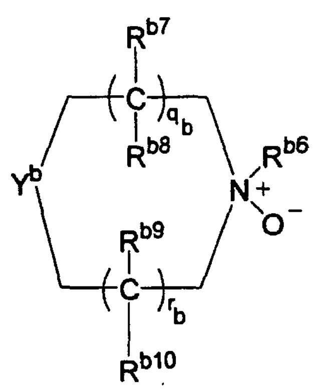

(b5-4)

(where, $R^{b1}$ represents a hydrogen atom or a methyl group, $R^{b2}$ and $R^{b3}$ represent a $C_{1-24}$ alkyl group or aryl group or a $C_{7-24}$ aralkyl group, which may be same or different each other; $R^{b4}$ and $R^{b5}$ represent a $C_{1-24}$ alkyl group, a $C_{6-24}$ aryl group or aralkyl group; $X^b$ represents a divalent linking group; $m_b$ is an integer of 0 or 1; $n_b$ is an integer from 0 to 4; $p_b$ is an integer from 0 to 3; and q and r represent an integer from 1 to 10, which may be same or different each other. $Y^b$ represents at least one divalent linking group selected from the group consisting of -C($R^{b11}$)($R^{b12}$)-, -N($R^{b13}$)-, -S- and -O-. At least one of $R^{b6}$ to $R^{b10}$, $R^{b11}$, $R^{b12}$ and $R^{b13}$ represents a double-bond-containing groups represented by $CH_2$=C($R^{b1}$)-($X^b$)$_{mb}$-, and other $R^{b6}$ to $R^{b10}$, $R^{b11}$, $R^{b12}$ and $R^{b13}$ respectively represent a hydrogen atom, a $C_{1-24}$ alkyl group, or a $C_{6-24}$ aryl group or aralkyl group.)

**22.** The hair cosmetic polymer of any one of claims 1 to 11, comprising, in addition to the copolymer (a), a silicone derivative (b6).

**23.** The hair cosmetic polymer of claim 22, wherein an amount of the copolymer (a) is within a range from 0.01 to 20% by weight of the total composition, and an amount of the silicone derivative (b6) is within a range from 0.01 to 50% by weight of the total composition.

**24.** A cosmetic comprising a composition as set forth in any one of claims 1 to 23.

**25.** The cosmetic of claim 24 for use on hair, skin or nail.

## INTERNATIONAL SEARCH REPORT

International application No.
PCT/JP02/09338

A. CLASSIFICATION OF SUBJECT MATTER
Int.Cl⁷ A61K7/00, 7/06, 7/04, C08F293/00

According to International Patent Classification (IPC) or to both national classification and IPC

B. FIELDS SEARCHED

Minimum documentation searched (classification system followed by classification symbols)
Int.Cl⁷ A61K7/00, 7/06, 7/04, C08F293/00

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | EP 766957 A1 (Wako Pure Chemical Industries, Ltd.),<br>09 April, 1997 (09.04.97),<br>Full text<br>& JP 9-157339 A & US 5840291 A<br>& KR 97020086 A & CN 1158720 A | 1-19,22-25<br>20,21 |
| X<br>Y | JP 2001-081018 A (Osaka Organic Chemical Ind. Co., Ltd.),<br>27 March, 2001 (27.03.01),<br>Full text (Family: none) | 1-19,22-25<br>20,21 |
| X<br>Y | EP 492657 A1 (Nippon Unicar Co., Ltd.),<br>01 July, 1992 (01.07.92),<br>Full text<br>& JP 4-211605 A & JP 4-234307 A<br>& US 5472686 A & US 5660819 A<br>& KR 158212 B | 1-19,22-25<br>20,21 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>16 December, 2002 (16.12.02) | Date of mailing of the international search report<br>14 January, 2003 (14.01.03) |
|---|---|
| Name and mailing address of the ISA/<br>Japanese Patent Office<br><br>Facsimile No. | Authorized officer<br><br><br>Telephone No. |

Form PCT/ISA/210 (second sheet) (July 1998)

**INTERNATIONAL SEARCH REPORT**

International application No.
PCT/JP02/09338

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | US 5851517 A (L'Oreal),<br>22 December, 1998 (22.12.98),<br>Full text; particularly, examples 1, 5, 7<br>& JP 10-501005 A & JP 2000-044426 A<br>& EP 749747 A1 & WO 97/00663 A1<br>& FR 2735689 A | 1-19,22-25<br>20,21 |
| Y | EP 754444 A2 (Mitsubishi Chemical Corp.),<br>22 January, 1997 (22.01.97),<br>Full text<br>& JP 10-072323 A & US 6123933 A<br>& DE 69600316 A & CN 1142935 A | 20,21 |
| P,X | JP 2001-288233 A (Shiseido Co., Ltd.),<br>16 October, 2001 (16.10.01),<br>Full text<br>(Family: none) | 1-25 |

Form PCT/ISA/210 (continuation of second sheet) (July 1998)

**INTERNATIONAL SEARCH REPORT**

International application No.
PCT/JP02/09338

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

The special technical feature of claims 1 to 10 resides in resin compositions for cosmetics containing a linear block copolymer which has a constitutional unit derived from a compound having an ethylenic unsaturated bond, has a number-average molecular weight of from $1.0x10^3$ to $1.0x10^6$ and has at least two glass transition points or melting points. The special technical feature of claim 11 resides in compositions for hair cosmetics containing a copolymer which is capable of forming a film having Young's modulus of 50 Mpa or above and an elongation at break of 100% or above, and dispersible in water and/or alcohols.

It does not appear that there is a technical (continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.

☐ No protest accompanied the payment of additional search fees.

Form PCT/ISA/210 (continuation of first sheet (1)) (July 1998)

INTERNATIONAL SEARCH REPORT

International application No.
PCT/JP02/09338

Continuation of Box No.II of continuation of first sheet(1)

relationship between these groups of inventions involving one or more of the same or corresponding special technical features. Such being the case, these two groups of inventions are not considered as relating to a group of inventions so linked as to form a single general inventive concept.

Form PCT/ISA/210 (extra sheet) (July 1998)